# EUROPÄISCHE PATENTSCHRIFT

(11) **EP 2 720 598 B1**
(45) Veröffentlichungstag und Bekanntmachung des Hinweises auf die Patenterteilung: **07.10.2015**
(21) Anmeldenummer: 12728221.8
(22) Anmeldetag: 13.06.2012
(51) Int. Cl.: A47L 15/00, A61L 2/18, A61L 2/22, D06F 33/02, D06F 39/00, A47L 15/24, A47L 15/42

(54) **REINIGUNGSVORRICHTUNG MIT ENERGIESPEICHER**
CLEANING DEVICE HAVING AN ENERGY STORE
DISPOSITIF DE NETTOYAGE À ACCUMULATEUR D'ÉNERGIE

(30) Priorität: 16.06.2011 DE 102011077660
(43) Veröffentlichungstag der Anmeldung: 23.04.2014
(73) Patentinhaber: MEIKO Maschinenbau GmbH & Co. KG, 77652 Offenburg (DE)
(72) Erfinder: GAUS, Bruno, 77654 Offenburg (DE); HILS, Wendelin, 77836 Rheinmünster (DE); PEUKERT, Thomas, 77815 Bühl (DE); SCHERINGER, Stefan, 77654 Offenburg (DE)
(74) Vertreter: Herzog, Fiesser & Partner Patentanwälte PartG mbB
(86) Internationale Anmeldenummer: PCT/EP2012/061168
(87) Internationale Veröffentlichungsnummer: WO 2012/171942

(56) Entgegenhaltungen:
- EP-A1- 1 088 927
- EP-A1- 1 253 696
- DE-A1-102007 014 425
- US-A1- 2003 025 395
- US-A1- 2009 313 767

## Beschreibung

### Gebiet der Erfindung

Die Erfindung betrifft eine Reinigungsvorrichtung und ein Verfahren zum Reinigen von Reinigungsgut. Derartige Reinigungsvorrichtungen und Verfahren werden in vielen Bereichen eingesetzt, um Reinigungsgut mit mindestens einem Reinigungsfluid zu beaufschlagen und dadurch zu reinigen. Beispiele einer Anwendung derartiger Reinigungsvorrichtungen sind die Geschirrspültechnik, sowohl im privaten Bereich als auch im gewerblichen Bereich, sowie die Reinigung von Gefäßen zur Aufnahme von menschlichen Ausscheidungen, wie beispielsweise Steckbecken, Bettpfannen oder ähnlichen Gefäßen, oder auch von anderen Arten von Pflegeutensilien, beispielsweise für Krankenhäuser oder Pflegeeinrichtungen.

### Stand der Technik

Aus dem Stand der Technik sind zahlreiche Reinigungsvorrichtungen bekannt, welche eingerichtet sind, um eine Reinigung von Reinigungsgut mittels mindestens eines Reinigungsfluids durchzuführen. Beispiele derartiger Reinigungsvorrichtungen sind in DE 10 2004 046 758 A1, in DE 10 2007 053 381 B3 oder auch in DE 10 2009 035 668 A1 dargestellt, wobei in diesen Dokumenten überwiegend auf Geschirrspülmaschinen Bezug genommen wird. Reinigungsgeräte zur Reinigung von Steckbecken, Bettpfannen oder ähnlichen Gefäßen zur Aufnahme menschlicher Ausscheidungen sind beispielsweise aus DE 103 48 344 A1 bekannt. Derartige Reinigungsgeräte werden häufig auch als Reinigungs- und Desinfektionsgeräte (RDGs) bezeichnet.

EP 1 088 927 A1 beschreibt ein intelligentes Dosierungsgerät zur Dosierung von Wirkstoffen. Das Dosiergerät umfasst eine wiederaufladbare Batterie, welche beispielsweise einen Aktor zur Dosierung des Wirkstoffs betreiben kann. Zum Wiederaufladen der Batterie umfasst das Gehäuse beispielsweise einen Stecker.

In DE 10 2007 014 425 A1 wird ein bewegliches Dosiersystem zur Abgabe von fließ- oder streufähigen Zubereitungen beschrieben. Dieses kann insbesondere für eine bedarfsgerechte Dosierung von Wirkstoffen in Geschirrspülmaschinen eingesetzt werden. Das Dosiersystem ist, beweglich in dem Sinne, dass dieses nicht unlösbar mit einer Vorrichtung, beispielsweise einer Geschirrspülmaschine, verbunden ist, sondern beispielsweise aus einer Geschirrspülmaschine entnehmbar oder in einer Geschirrspülmaschine positionierbar ist. Das Dosiergerät umfasst eine Energiequelle, eine Sensoreinheit, eine Steuereinheit sowie mindestens einen Aktuator oder Spender. Die Energiequelle kann beispielsweise einen Akkumulator umfassen, welcher beispielsweise induktiv wiederaufladbar ist. Alternativ können auch mechanische Energiequellen vorgesehen sein.

US 2009/0313767 A1 beschreibt ein schnurloses Dampfgerät mit einem integrierten Dampfgenerator. Dieser umfasst austauschbare Batterien.

US 2003/0025395 A1 beschreibt eine transformatorlose Energiesteuerung für Vorrichtungen wie beispielsweise Waschmaschinen. Das Dokument beschreibt, neben der regulären Energieversorgung, die Verwendung eines Niederenergie-Schaltkreises, welcher verwendet wird, um Energie für Standby- oder Niederenergie-Komponenten bereitzustellen. Dabei werden Kondensatoren verwendet, um die niedrige Energie bereitzustellen.

Reinigungsgeräte wie Spülmaschinen oder RDGs weisen üblicherweise einen oder mehrere elektrische Verbraucher auf. Beispielsweise können diese Verbraucher einen oder mehrere Motoren oder eine oder mehrere Heizungen, beispielsweise in Form von Boilern oder Durchlauferhitzern, aufweisen. Diese Verbraucher können, je nach Erfordernis der ablaufenden Prozesse, einzeln oder in beliebiger Kombination eingeschaltet werden, beispielsweise entsprechend einem Programmablauf des Reinigungsgeräts. Hierbei tritt, je nach Programmablauf, ein teilweise recht erheblicher elektrischer Energiebedarf auf.

Dementsprechend gewinnt, insbesondere im Bereich der gewerblichen Geschirrspültechnik, jedoch auch im Bereich der Reinigungs- und Desinfektionsgeräte sowie im Bereich der Haushaltstechnik, die Einsparung von Energie zunehmend an Bedeutung. So werden beispielsweise in DE 10 2004 046 758 A1 ein Verfahren und eine Anordnung zum energiesparenden Betrieb von Spülmaschinen beschrieben. Dabei wird einer Gruppe von elektrischen Verbraucherelementen einer Spülmaschine eine maximale elektrische Gesamtleistung zugewiesen. In einem Bedarfsermittlungsschritt wird, abhängig von einem Betriebszustand der Spülmaschine, eine optimale Kombination von Leistungsleveln gewählt, wobei für jedes Verbraucherelement der Spülmaschine das gewählte Leistungslevel dem Leistungsbedarf des Verbraucherelements in dem Betriebszustand angepasst ist und wobei die aufgenommene Gesamtleistung aller Verbraucherelemente die zulässige maximale elektrische Gesamtleistung nicht überschreitet.

Aus DE 10 2007 053 381 B3 ist eine Geschirrspülmaschine mit einem Latentwärmespeicher bekannt. Dabei wird eine Wärmerückgewinnungseinrichtung verwendet, welche einer feuchten Luft aus einer Reinigungskammer des Reinigungsgeräts Wärme entzieht und dem Reinigungsgerät über ein erstes Kühlfluid wieder zuführt. Das Reinigungsgerät weist eine Kühlfluidleitung mit mindestens einem Rückführpfad sowie mindestens einem Wärmespeicherpfad mit mindestens einem Latentwärmespeicher auf. Das Reinigungsgerät ist eingerichtet, um das erste Kühlfluid in mindestens einem Betriebszustand nach Durchströmen der Wärmerückgewinnungseinrichtung über den Rückführpfad zu der Reinigungskammer und/oder in einen Fluidtank zu leiten. Das Reinigungsgerät ist weiterhin eingerichtet, um das erste Kühlfluid in mindestens einem Standby-Betrieb über den Wärmespeicherpfad dem Latentwärmespeicher zuzuführen.

Aus DE 10 2009 035 668 A1 ist eine Reinigungsvorrichtung zur Reinigung von Reinigungsgut bekannt. Die Reinigungsvorrichtung umfasst mindestens zwei Verbraucher. Die Reinigungsvorrichtung weist eine modulare Steuerung auf, welche eine Maschinensteuerung und mindestens ein kontaktloses Steuerelement umfasst. Dabei kann mindestens ein Reinigungsprogramm durchgeführt werden, wobei das kontaktlose Steuerelement mindestens einen der Verbraucher variabel mit Energie versorgt. Die Maschinensteuerung kann mindestens zwei verschiedene Ansteuerstrategien für die Reinigungsvorrichtung bereitstellen, wobei jede Ansteuerstrategie Informationen über benötigte Energien der Verbraucher umfasst. Die Maschinensteuerung kann dem kontaktlosen Steuerelement über mindestens ein Bus-System Informationen über die benötigten Energien der zugehörigen Verbraucher entsprechend einer ausgewählten Ansteuerstrategie übermitteln. Das kontaktlose Steuerelement kann mindestens einen zugehörigen Verbraucher mit der jeweils benötigten Energie beaufschlagen.

Trotz der Vorteile, welche sich aus den bekannten Energiesparkonzepten ergeben, kann nach wie vor eine erhebliche Strom- und/oder Leistungsaufnahme aus einem bauseitigen elektrischen Versorgungsnetz auftreten. Sind beispielsweise alle Verbraucher gleichzeitig eingeschaltet, so kann die aufgenommene Leistung beispielsweise bei üblichen Programmautomaten, also gewerblichen Geschirrspülmaschinen mit stationärem Spülverfahren, 3 kW bis 12 kW betragen. Bei Transportgeschirrspülmaschinen kann die Leistungsaufnahme typischerweise maximal 15 kW bis 70 kW betragen. Bei Reinigungs- und Desinfektionsgeräten sind üblicherweise maximale Leistungsaufnahmen bei gleichzeitiger Einschaltung sämtlicher Verbraucher von 4 kW bis 5kW zu verzeichnen.

Elektrische Zuleitungen der Reinigungsgeräte müssen bauseitig auf maximal auftretende elektrische Leistungsaufnahmen ausgelegt werden, beispielsweise auf die genannten Werte. Zudem muss der Betreiber der Reinigungsvorrichtung üblicherweise vertraglich mit seinem Stromversorger absichern, dass die entsprechende Leistungsbereitstellung und Leistungsabgabe gewährleistet ist. Die Kosten, sowohl für die bauseitigen Installationen als auch für den Umfang des Stromliefervertrags mit dem Energieversorger, steigen in der Regel mit der Höhe der maximal aufgenommenen Leistung. Zwar sind beispielsweise die in DE 10 2004 046 758 A1 oder in DE 10 2009 035 668 A1 beschriebenen, fest eingerichteten Verriegelungen in der Regel dazu in der Lage, sicherzustellen, dass nicht alle Verbraucher gleichzeitig eingeschaltet werden. Weiterhin kann mittels fest eingerichteter Verriegelungen oder auch variabler Verriegelungen eine Steuerung des Reinigungsgeräts derart eingestellt werden, dass die aufgenommene Leistung variabel je nach Bedarf den einzelnen Verbrauchern zugeordnet wird. Dennoch besteht nach wie vor ein erhebliches Verbesserungspotential und Optimierungspotential, insbesondere hinsichtlich hoher maximaler aufgenommener Leistungen. Diese maximalen Leistungen verursachen für den Betreiber in der Regel hohe Kosten, sowohl bei der elektrischen Installation als auch bei der Vertragsgestaltung mit den entsprechenden Energieversorgern. Auch unter Umweltaspekten sind derartige hohe maximale Leistungen ungünstig. Viele der bekannten Strategien zur Begrenzung der aufgenommenen Leistungen begrenzen naturgemäß auch die Leistung des Reinigungsgeräts, in der diese Strategien zum Einsatz kommen.

### Aufgabe der Erfindung

Es ist daher eine Aufgabe der vorliegenden Erfindung, eine Reinigungsvorrichtung und ein Verfahren zum Reinigen von Reinigungsgut bereitzustellen, welche die Nachteile bekannter Vorrichtungen und Verfahren dieser Art zumindest weitgehend vermeiden. Insbesondere soll gewährleistet werden, dass eine maximale elektrische Leistungsaufnahme der Reinigungsgeräte gesenkt werden kann, ohne dass hierdurch die Leistung des Reinigungsgeräts erheblich beeinträchtigt wird.

### Offenbarung der Erfindung

Diese Aufgabe wird gelöst durch eine Reinigungsvorrichtung und ein Verfahren zum Reinigen von Reinigungsgut mit den Merkmalen der unabhängigen Patentansprüche. Vorteilhafte Weiterbildungen, welche einzeln oder in beliebiger Kombination verwendet werden können, sind in den abhängigen Ansprüchen dargestellt.

Allgemein wird darauf hingewiesen, dass die Begriffe "aufweisen" und "umfassen" sowie entsprechende grammatikalische Abwandlungen dieser Begriffe im Rahmen der vorliegenden Erfindung sowohl abschließend als auch nicht-abschließend verstanden werden können. So können der Ausdruck "A weist B auf" oder der Ausdruck "A umfasst B" sowohl bedeuten, dass A ausschließlich aus einem oder mehreren B besteht als auch, dass A neben einem oder mehreren B mindestens ein weiteres Element aufweist.

In einem ersten Aspekt der vorliegenden Erfindung wird eine Reinigungsvorrichtung zum Reinigen von Reinigungsgut vorgeschlagen. In einem zweiten Aspekt der vorliegenden Erfindung wird ein Verfahren zum Reinigen von Reinigungsgut vorgeschlagen. Dabei kann die Reinigungsvorrichtung eingerichtet sein, um das erfindungsgemäße Verfahren durchzuführen. Das Verfahren wird unter Verwendung einer erfindungsgemäßen Reinigungsvorrichtung durchgeführt. Dementsprechend kann bezüglich möglicher Ausgestaltungen der Reinigungsvorrichtung auf die Beschreibung optionaler Ausgestaltungen des Verfahrens verwiesen werden und umgekehrt.

Beispielsweise kann die Reinigungsvorrichtung, wie unten noch näher erläutert wird, mindestens eine Steuerung aufweisen, beispielsweise eine zentrale oder dezentrale Maschinensteuerung, welche, beispielsweise programmtechnisch, eingerichtet sein kann, um das Verfahren umzusetzen. Beispielsweise kann diese Maschinensteuerung mindestens eine Datenverarbeitungsvorrichtung umfassen, optional mit einem oder mehreren flüchtigen und/oder nicht flüchtigen Datenspeichern, wobei die Datenverarbeitungsvorrichtung beispielsweise programmtechnisch eingerichtet sein kann, um die Reinigungsvorrichtung zur Durchführung eines erfindungsgemäßen Verfahrens anzusteuern. Beispielsweise kann die Datenverarbeitungsvorrichtung eingerichtet sein, um mindestens ein Reinigungsprogramm mit mindestens zwei Reinigungsprogrammschritten durchzuführen. Weiterhin kann, alternativ oder zusätzlich, die Steuerung eingerichtet sein, um die Reinigungsvorrichtung in mindestens zwei verschiedenen Betriebszuständen zu steuern, was unten noch näher erläutert wird.

Die Reinigungsvorrichtung und das Verfahren dienen zum Reinigen von Reinigungsgut. Bei diesem Reinigungsgut kann es sich grundsätzlich um beliebiges Gut handeln, welches mittels eines Reinigungsfluids, also eines gasförmigen oder flüssigen Mediums, gereinigt werden kann. Insbesondere kann das Reinigungsgut Geschirr und/oder andere Gegenstände umfassen, welche zur Zubereitung, Lagerung oder Darreichung von Speisen und Getränken eingerichtet sind. So kann das Geschirr insbesondere ausgewählt sein aus der Gruppe bestehend aus: Tassen, Tellern, Besteck, Gläsern, Bechern, Schüsseln, Töpfen und Tabletts. Alternativ oder zusätzlich kann das Reinigungsgut auch aus anderen Bereichen stammen, insbesondere dem Bereich der Medizintechnik und/oder der Kranken- und Altenpflege. So kann das Reinigungsgut insbesondere ein oder mehrere Gefäße zur Aufnahme menschlicher oder tierischer Ausscheidungen umfassen, insbesondere Nachtgeschirr, Bettpfannen, Steckbecken und Urinflaschen, insbesondere Gefäßen mit einem Aufnahmevolumen von mindestens 100 ml, vorzugsweise mindestens 500 ml. Wiederum alternativ oder zusätzlich kann das Reinigungsgut beispielsweise andere medizinische Geräte und/oder Maschinenteile umfassen.

Unter einer Reinigung wird allgemein im Rahmen einer vorliegenden Erfindung ein Verfahren verstanden, bei welchem ein Gegenstand ganz oder teilweise von Verunreinigungen befreit wird, wobei diese Verunreinigungen organischer und/oder anorganischer Natur sein können und wobei diese Verunreinigungen insbesondere auch eine oder mehrere Arten von Mikroorganismen umfassen können. Insbesondere können diese Verunreinigungen an dem Gegenstand anhaftende Verunreinigungen und/oder in mindestens einem Hohlraum des Gegenstands enthaltene Verunreinigungen sein. Die Verunreinigungen können insbesondere Speisereste und/oder menschliche oder tierische Ausscheidungen umfassen. Die Reinigung kann weiterhin, alternativ oder zusätzlich, auch eine vollständige oder teilweise Entkeimung umfassen, also eine vollständige oder teilweise Befreiung des Gegenstands von Mikroorganismen, insbesondere eine Desinfektion und/oder Sterilisation.

Das Verfahren wird derart durchgeführt, dass das Reinigungsgut mittels mindestens einer Fluidvorrichtung mit mindestens einem Reinigungsfluid beaufschlagt wird, und die Reinigungsvorrichtung umfasst mindestens eine Fluidvorrichtung zum Beaufschlagen des Reinigungsguts mit mindestens einem Reinigungsfluid. Unter einem Reinigungsfluid kann ein flüssiges und/oder gasförmiges Medium verstanden werden, welches eingerichtet ist, um anhaftende Verunreinigungen von dem Reinigungsgut zu entfernen und/oder um das Reinigungsgut zu desinfizieren oder sogar zu sterilisieren. Insbesondere kann das Reinigungsfluid mindestens ein wässriges Reinigungsfluid umfassen, insbesondere eine Spülflüssigkeit, welche Wasser sowie einen oder mehrere Zusätze mindestens eines Reinigers und/oder mindestens eines Klarspülers und/oder mindestens eines Desinfektionsmittels umfasst. Weiterhin kann, alternativ oder zusätzlich, das Reinigungsfluid auch beispielsweise eine oder mehrere nichtwässrige Medien und/oder ein oder mehrere gasförmige Medien umfassen. Alternativ oder zusätzlich kann das Reinigungsfluid mindestens einen Dampf umfassen, insbesondere zur Desinfektion und/oder Sterilisation des Reinigungsguts. Insbesondere kann das Reinigungsfluid Heißdampf umfassen, also Wasserdampf, optional unter Zusatz eines oder mehrerer Hilfsstoffe wie beispielsweise mindestens einem Desinfektionsmittel.

Unter einer Fluidvorrichtung ist grundsätzlich eine beliebige Vorrichtung zu verstehen, welche zur Beaufschlagung des Reinigungsguts mit dem Reinigungsfluid eingerichtet ist. Insbesondere kann die Fluidvorrichtung mindestens eine Öffnung aufweisen, an welcher das Reinigungsfluid, beispielsweise über mindestens eine Versorgungsleitung, bereitgestellt wird und aus welcher das Reinigungsfluid austreten kann. Insbesondere kann die Fluidvorrichtung mindestens eine Düse und/oder mindestens ein Düsensystem aufweisen, aus welchem das Reinigungsfluid austreten kann, beispielsweise strahlförmig, um auf das Reinigungsgut aufzutreffen. Beispielsweise kann die Fluidvorrichtung einen oder mehrere Düsenarme und/oder ein oder mehrere Düsensysteme aufweisen.

Die Reinigungsvorrichtung weist weiterhin mindestens einen elektrischen Verbraucher auf, und das Verfahren wird derart durchgeführt, dass bei dem Verfahren mindestens ein elektrischer Verbraucher verwendet wird. Unter einem elektrischen Verbraucher ist allgemein eine Vorrichtung zu verstehen, welche eingerichtet ist, um elektrische Energie aufzunehmen und diese beispielsweise umzusetzen in andere Energieformen und/oder in Rechenleistung und/oder in gespeicherte Informationen. Insbesondere kann der mindestens eine Verbraucher eingerichtet sein, um elektrische Energie in mechanische Energie, beispielsweise Bewegungsenergie und/oder Druck, umzuwandeln. Alternativ oder zusätzlich kann der mindestens eine Verbraucher eingerichtet sein, um die elektrische Energie ganz oder teilweise in Wärmenergie umzuwandeln, beispielsweise zur Erwärmung des mindestens einen Reinigungsfluids und/oder zur Erwärmung des Reinigungsguts.

Beispielsweise kann der mindestens eine Verbraucher ausgewählt sein aus der Gruppe bestehend aus: einer Erwärmungsvorrichtung, insbesondere einem Boiler, einer Heizspirale oder einem Durchlauferhitzer; einem Motor, insbesondere einem Motor zum Antrieb eines Transportsystems für das Reinigungsgut und/oder einem Motor zum Antrieb einer Bewegung der Fluidvorrichtung; einer Steuerung zum Steuern der Reinigungsvorrichtung, insbesondere einer Datenverarbeitungsvorrichtung und/oder einem Datenspeicher; einer Pumpe, insbesondere einer Pumpe zum Befördern des Reinigungsfluids und/oder einer Pumpe zur Beaufschlagung des Reinigungsguts mit dem Reinigungsfluid; einem Gebläse, insbesondere einem Gebläse zur Absaugung von Warmluft aus einer Reinigungskammer der Reinigungsvorrichtung und/oder einem Gebläse zur Beaufschlagung des Reinigungsguts mit mindestens einem gasförmigen Medium, insbesondere einem Warmluftgebläse. Auch andere Ausgestaltungen der Verbraucher sowie Kombinationen der genannten Optionen und/oder anderer Optionen sind möglich.

Die Reinigungsvorrichtung weist weiterhin mindestens einen elektrischen Anschluss zur Versorgung der Reinigungsvorrichtung mit elektrischer Energie auf, und das Verfahren ist derart ausgestaltet, dass mindestens ein elektrischer Anschluss zur Versorgung des Verfahrens mit elektrischer Energie verwendet wird. Unter einem elektrischen Anschluss ist grundsätzlich eine beliebige Vorrichtung zu verstehen, welche elektrische Energie an die Reinigungsvorrichtung bzw. zur Verwendung in dem Verfahren bereitstellen kann. Insbesondere kann es sich hierbei um einen festen gebäudeseitigen Anschluss handeln, beispielsweise einen festverdrahteten Gebäudeanschluss mit mindestens einem Energieversorgungskabel. Auch eine lösbare Ausgestaltung des elektrischen Anschlusses ist jedoch grundsätzlich denkbar, beispielsweise in Form mindestens eines männlichen und/oder weiblichen Steckers, der beispielsweise mit mindestens einem gebäudeseitigen Stecker und/oder mindestens einem Kabel verbindbar ist. Grundsätzlich ist alternativ oder zusätzlich zu einem kabelgebundenen Anschluss auch eine drahtlose Energieversorgung denkbar. Beispielsweise kann der Anschluss ausgestaltet sein, um eine elektrische Leistung von mindestens 200 W, insbesondere mindestens 1 kW und besonders bevorzugt mindestens 5 kW von mindestens einer Energieversorgung aufzunehmen und an die Reinigungsvorrichtung bzw. das Verfahren bereitzustellen.

Die Reinigungsvorrichtung weist mindestens einen Energiespeicher auf, und das Verfahren wird derart durchgeführt, dass mindestens ein Energiespeicher verwendet wird. Unter einem Energiespeicher ist grundsätzlich im Rahmen der vorliegenden Erfindung eine beliebige Vorrichtung zu verstehen, welche eingerichtet ist, um Energie aufzunehmen, zu speichern und diese Energie wieder abzugeben. Beispiele derartiger Energiespeicher werden unten noch näher erläutert. Der Energiespeicher kann insbesondere als elektrischer Puffer wirken und/oder als elektrischer Puffer eingerichtet sein, also als Vorrichtung, welche für eine Zwischenspeicherung elektrischer Energie geeignet oder eingerichtet ist.

Die Reinigungsvorrichtung und das Verfahren sind weiterhin derart eingerichtet, sodass über den elektrischen Anschluss elektrische Energie aufgenommen und in dem Energiespeicher zwischengespeichert wird. Unter einer Zwischenspeicherung ist dabei allgemein eine Speicherung von Energie zu verstehen, welche dem Zweck einer späteren Verwendung der Energie dient. Dabei kann die über den elektrischen Anschluss aufgenommene elektrische Energie, wie unten noch näher ausgeführt wird, in Form elektrischer Energie in dem Energiespeicher zwischengespeichert werden und/oder in mindestens einer anderen Energieform, beispielsweise in Form von Wärme und/oder in Form von chemischer Energie und/oder in Form von mechanischer Energie.

Die Reinigungsvorrichtung und das Verfahren sind weiterhin derart eingerichtet, dass der mindestens eine elektrische Verbraucher zumindest teilweise mit elektrischer Energie aus dem Zwischenspeicher versorgt wird, insbesondere in mindestens einer Betriebsphase und/oder in mindestens einem Programmschritt. Insbesondere können die Reinigungsvorrichtung und das Verfahren derart ausgestaltet sein, dass elektrische Energie über den elektrischen Anschluss aufgenommen und in dem Energiespeicher zwischengespeichert werden kann, bevor die Energie erstmalig einem oder mehreren elektrischen Verbrauchern zugeführt wird.

Die Energieversorgung des mindestens einen elektrischen Verbrauchers mit elektrischer Energie aus dem mindestens einen Energiespeicher kann direkt erfolgen, sodass die in dem Energiespeicher gespeicherte Energie direkt dem elektrischen Verbraucher zugeführt wird und/oder indirekt, beispielsweise nach Umwandlung der in dem Energiespeicher aufgenommenen Energie in mindestens eine andere Energieform. So kann, wie oben ausgeführt, die Energie in dem Zwischenspeicher auch in nicht-elektrischer Form zwischengespeichert sein. In diesem Fall sollte, wie unten exemplarisch noch näher ausgeführt wird, diese zwischengespeicherte Energie zunächst in elektrische Energie umgewandelt werden, bevor diese elektrische Energie dann dem mindestens einen elektrischen Verbraucher zugeführt wird. Sowohl das Zwischenspeichern der aus dem elektrischen Anschluss aufgenommenen elektrischen Energie als auch die Versorgung des elektrischen Verbrauchers mit elektrischer Energie aus dem Zwischenspeicher können also direkt und/oder indirekt erfolgen, also indem die Zwischenspeicherung in Form elektrischer Energie und/oder in Form nicht-elektrischer Energie, letzteres mit entsprechender Umwandlung zwischen den Energieformen, erfolgt.

Wie oben ausgeführt, kann die Reinigungsvorrichtung insbesondere ausgewählt sein aus der Gruppe bestehend aus: einer Geschirrspülmaschine, insbesondere einem Programmautomaten und/oder einer Durchlauf-Geschirrspülmaschine, insbesondere einer Geschirrspülmaschine für den gewerblichen Einsatz; einem Reinigungs- und Desinfektionsgerät, beispielsweise zur Reinigung von Ausscheidungsgefäßen zur Aufnahme menschlicher Ausscheidungen und/oder zur Reinigung anderer Arten von medizinischem Gerät. Die Geschirrspülmaschine kann beispielsweise als Einkammer-Geschirrspülmaschine oder auch als Mehrkammer-Geschirrspülmaschine ausgestaltet sein. Unter einem Programmautomaten kann im Rahmen der vorliegenden Erfindung insbesondere eine Geschirrspülmaschine mit einem stationärem Spülverfahren verstanden werden, also eine Geschirrspülmaschine, bei welcher das Reinigungsgut während der Reinigung in einer Reinigungskammer verbleibt, wobei mehrere Behandlungsschritte nacheinander im Rahmen eines Reinigungsprogramms durchgeführt werden können. Unter einer gewerblichen Geschirrspülmaschine, welche im Rahmen der vorliegenden Erfindung besonders bevorzugt ist, wird allgemein eine Geschirrspülmaschine verstanden, welche mindestens zwei Tanks zur getrennten Aufnahme und/oder Aufbereitung von Reinigungsfluid aufweist und welche eingerichtet ist, um größere Mengen an Geschirr mit hohem Durchsatz zu reinigen. Bezüglich möglicher Ausgestaltungen der Einkammer-Geschirrspülmaschine und/oder der Durchlauf-Geschirrspülmaschine kann beispielsweise auf den oben zitierten Stand der Technik verwiesen werden, welche erfindungsgemäß abgewandelt werden können. Das Reinigungs- und Desinfektionsgerät kann allgemein eingerichtet sein, um Gefäße zu reinigen, welche zur Aufnahme größerer Abfallmengen eingerichtet sind, beispielsweise Abfallmengen flüssiger und/oder fester Ausscheidungen mit einer Menge von mindestens 100 ml, insbesondere mindestens 200 ml oder sogar mindestens 500 ml oder mindestens 1 1 oder mindestens 2 1. Dementsprechend kann das Reinigungs- und Desinfektionsgerät beispielsweise über mindestens einen Abfluss verfügen, welcher zur Abfuhr der genannten Flüssigkeitsmengen eingerichtet ist, beispielsweise einen Abfluss mit einem Siphonbogen. Für mögliche Ausgestaltungen des Reinigungs- und Desinfektionsgeräts kann beispielsweise auf die oben zitierte DE 103 48 344 A1 verwiesen werden, wobei das dort beschriebene Reinigungs- und Desinfektionsgerät erfindungsgemäß abgewandelt werden kann.

Allgemein weist die Reinigungsvorrichtung mindestens eine Reinigungskammer auf, in welcher die Beaufschlagung des Reinigungsguts mit dem Reinigungsfluid erfolgt. Die Kammer kann offen ausgestaltet sein und beispielsweise lediglich einen Austritt größerer Flüssigkeitsmengen oder größerer Dampfmengen in eine Umgebung verhindern, oder kann geschlossen ausgestaltet sein, so dass ein Austritt von Flüssigkeit und/oder Dampf aus der Kammer in eine Arbeitsumgebung vollständig verhindert wird. Es können eine oder auch mehrere Kammern vorgesehen sein. Die mindestens eine Kammer kann beispielsweise durch mindestens einen Verschluss verschließbar sein, beispielsweise mindestens eine Beladeklappe. Alternativ oder zusätzlich kann die mindestens eine Kammer jedoch auch beispielsweise mindestens einen Tunnel aufweisen, wie beispielsweise bei den oben beschriebenen Durchlaufgeschirrspülmaschinen gemäß dem Stand der Technik.

Weitere optionale Ausgestaltungen betreffen eine Steuerung der Energieaufnahme und/oder eine Steuerung der Versorgung des mindestens einen elektrischen Verbrauchers mit elektrischer Energie. Die im Folgenden beschriebenen Maßnahmen können beispielsweise apparativ dadurch umgesetzt werden, dass, wie oben ausgeführt, die Reinigungsvorrichtung mindestens eine Steuerung aufweist, beispielsweise eine Steuerung mit mindestens einer Datenverarbeitungsvorrichtung, welche zur Durchführung der beschriebenen Ausgestaltungen eingerichtet ist. Entsprechend kann das vorgeschlagene Verfahren Verfahrensschritte zur Umsetzung der optionalen Merkmale aufweisen.

Die Reinigungsvorrichtung ist eingerichtet, um den mindestens einen elektrischen Verbraucher wahlweise, beispielsweise nach Vorgabe durch die Steuerung, mit elektrischer Energie aus dem Energiespeicher und wahlweise direkt mit über den elektrischen Anschluss aufgenommener elektrischer Energie zu versorgen. Beispielsweise kann die Reinigungsvorrichtung mindestens ein Schaltelement aufweisen, welches zwischen einer Versorgung des elektrischen Verbrauchers mit elektrischer Energie aus dem Energiespeicher und einer Versorgung des elektrischen Verbrauchers mit elektrischer Energie aus dem elektrischen Anschluss ohne Zwischenspeicherung in dem Energiespeicher stufenlos oder in mehreren Stufen umschalten kann. Unter einer stufenlosen Umschaltung kann beispielsweise ein Schalten zwischen einem Zustand, in welchem der elektrische Verbraucher ausschließlich mit elektrischer Energie aus dem Energiespeicher versorgt wird und einem Zustand, in welchem der elektrische Verbraucher ausschließlich mit elektrischer Energie aus dem elektrischen Anschluss ohne Zwischenspeicherung in dem Energiespeicher versorgt wird, verstanden werden. Unter einem Umschalten in einer oder mehrere Stufen kann beispielsweise ein Schalten verstanden werden, bei welchem zusätzlich zu den beiden genannten Zuständen mindestens ein weiterer Zustand existiert, in welchem der elektrische Verbraucher sowohl mit elektrischer Energie aus dem elektrischen Anschluss ohne Zwischenspeicherung in dem Energiespeicher als auch mit elektrischer Energie aus dem

Energiespeicher versorgt wird. Die stufenlose Schaltung kann realisiert werden, indem beispielsweise ein Mischungsverhältnis der Energieversorgung des elektrischen Verbrauchers mit elektrischer Energie aus dem elektrischen Anschluss ohne Zwischenspeicherung in dem Energiespeicher und mit elektrischer Energie aus dem Energiespeicher variabel einstellbar ist, insbesondere stufenlos, beispielsweise in einem vorgegebenen Mischungsverhältnisbereich. Sind mehrere elektrische Verbraucher vorgesehen, so kann das genannte optionale mindestens eine Schaltelement, welches die Art und/oder das Mischungsverhältnis der Energieversorgung des elektrischen Verbrauchers einstellen kann, für einen einzelnen dieser elektrischen Verbraucher, für mehrere ausgewählte dieser elektrischen Verbraucher oder auch für alle elektrischen Verbraucher gemeinsam vorgesehen sein, um die Energieversorgung einzustellen. Es können auch mehrere Schaltelemente der genannten Art vorgesehen sein, welche jeweils für einen oder mehrere elektrische Verbraucher in der genannten Art die Energieversorgung einstellen können.

Beispielsweise kann der elektrische Anschluss mit dem Energiespeicher direkt oder indirekt elektrisch verbunden sein, und der Energiespeicher kann direkt oder indirekt mit dem mindestens einen elektrischen Verbraucher verbunden sein. Zusätzlich kann optional beispielsweise der elektrische Anschluss direkt oder indirekt mit dem elektrischen Verbraucher verbunden sein, beispielsweise über mindestens eine elektrische Umgehungsleitung, welche den Energiespeicher umgeht. Die Reinigungsvorrichtung kann beispielsweise, wie oben beschrieben, mindestens ein Schaltelement, beispielsweise einen Schalter und/oder eine andere Art einer Vorrichtung aufweisen, welche einen Fluss der Energie über den Energiespeicher und/oder unter Umgehung des Energiespeichers hin zu dem mindestens einen elektrischen Verbraucher steuert und/oder regelt. Beispielsweise kann diese Vorrichtung mindestens ein Schaltelement aufweisen, welches zur Beeinflussung der Energieströme über die genannten Zweige eingerichtet sein kann.

Die Reinigungsvorrichtung ist weiterhin, wie oben bereits ausgeführt, eingerichtet, um den elektrischen Verbraucher gleichzeitig mit elektrischer Energie aus dem Energiespeicher und mit direkt über den elektrischen Anschluss aufgenommener elektrischer Energie zu versorgen. Auch dies kann beispielsweise unter Verwendung des mindestens einen optionalen Schaltelements erfolgen. So kann beispielsweise eine Ausgestaltung realisiert werden, bei welcher ein oder mehrere Verbraucher gleichzeitig sowohl mit Energie, welche zuvor in dem Energiespeicher zwischengespeichert war, als auch mit direkt von dem elektrischen Anschluss aufgenommener elektrische Energie versorgt werden. Sind mehrere Verbraucher vorgesehen, so kann beispielsweise die Reinigungsvorrichtung auch derart ausgestaltet sein, dass ein oder mehrere Verbraucher mit direkt über den elektrischen Anschluss aufgenommener Energie versorgt werden, ohne Zwischenspeicherung in dem Energiespeicher, wohingegen ein oder mehrere weitere Verbraucher alternativ oder zusätzlich mit elektrischer Energie aus dem Energiespeicher versorgt werden. Die Reinigungsvorrichtung kann insbesondere eingerichtet sein, um ein Verhältnis zwischen der elektrischen Energie aus dem Energiespeicher und der direkt über den elektrischen

Anschluss aufgenommenen elektrischen Energie ohne Zwischenspeicherung in dem Energiespeicher, mit der der elektrische Verbraucher beaufschlagt wird, zu verändern. Beispielsweise kann dieses Verhältnis bei einer Mischung einer Beaufschlagung mit elektrischer Energie aus dem Energiespeicher und elektrischer Energie aus dem elektrischen Anschluss stufenlos oder in einer oder mehreren verschiedenen Stufen variabel ausgestaltet sein. Insbesondere kann die Reinigungsvorrichtung eingerichtet sein, um das Verhältnis an einen aktuellen Betriebszustand anzupassen.

Die Reinigungsvorrichtung kann weiterhin insbesondere eingerichtet sein, um in mindestens zwei verschiedenen Betriebszuständen betrieben zu werden. Beispielsweise kann die Reinigungsvorrichtung, wie oben ausgeführt, mindestens eine Steuerung aufweisen, welche eingerichtet ist, um mindestens zwei Betriebszustände für die Reinigungsvorrichtung einzustellen.

Unter einem Betriebszustand ist dabei allgemein ein Zustand der Reinigungsvorrichtung zu verstehen, welcher durch einen Satz von Betriebsparametern der Reinigungsvorrichtung gekennzeichnet ist. Beispielsweise können diese Betriebsparameter für mehrere oder alle Elemente der Reinigungsvorrichtung, welche unterschiedliche Zustände annehmen können, den aktuell eingenommenen Zustand beschreiben. Hierbei kann es sich beispielsweise um elektrische und/oder mechanische und/oder um thermische Zustände eines oder mehrerer Elemente der Reinigungsvorrichtung und/oder des Reinigungsfluids und/oder des Reinigungsguts handeln. Beispielsweise können diese Betriebsparameter Drehzahlen von Motoren und/oder Pumpen, aktuelle elektrische Leistungsaufnahmen, Geschwindigkeit in einer Transportvorrichtung, Leistungsabgaben von Heizvorrichtungen, aktuelle Temperaturen eines oder mehrerer Reinigungsfluide, Umgebungstemperaturen in einer Reinigungskammer der Reinigungsvorrichtung oder ähnliche Betriebsparameter umfassen. Die Reinigungsvorrichtung kann, beispielsweise über die mindestens eine Steuerung, eingerichtet sein, um mehrere oder alle diese Betriebsparameter aktiv zu beeinflussen und/oder um diese Parameter zu erfassen.

Insbesondere kann die Reinigungsvorrichtung derart eingerichtet sein, dass in den mindestens zwei verschiedenen Betriebszuständen der mindestens eine Verbraucher eine unterschiedliche elektrische Leistungsaufnahme aufweist. Beispielsweise kann ein erster Betriebszustand existieren, in welchem der Verbraucher eine erste elektrische Leistungsaufnahme aufweist, und ein zweiter Betriebszustand, in welchem der Verbraucher eine zweite, von der ersten verschiedene elektrische Leitungsaufnahme aufweist.

Dabei können auch mindestens zwei verschiedene Betriebszustände vorgesehen sein, in welchen der Energiespeicher unterschiedlich genutzt wird. So kann mindestens ein erster Betriebszustand vorgesehen sein, in welchem der Energiespeicher aufgeladen wird, und mindestens ein zweiter Betriebszustand, in welchem der Energiespeicher entladen wird. So kann die Reinigungsvorrichtung insbesondere eingerichtet sein, dass in mindestens einem ersten Betriebszustand elektrische Energie über den elektrischen Anschluss aufgenommen wird und der Energiespeicher mit elektrischer Energie aufgeladen wird. Weiterhin kann in mindestens einem zweiten Betriebszustand elektrische Energie aus dem Energiespeicher entnommen und dem elektrischen Verbraucher zugeführt werden. Auch Mischformen sind möglich, beispielsweise Mischformen, in welchen gleichzeitig eine Aufladung des Energiespeichers und/oder mindestens eines Energiespeichers und eine Entnahme elektrischer Energie aus dem Energiespeicher und/oder aus mindestens einem Energiespeicher erfolgt.

Der erste Betriebszustand, in welchem eine Aufladung des Energiespeichers erfolgt, kann insbesondere auch einen Stillstand der Reinigungsvorrichtung umfassen, wobei in dem Stillstand keine Beaufschlagung des Reinigungsguts mit dem Reinigungsfluid erfolgt. So kann dieser Stillstand beispielsweise einen Standby-Betrieb umfassen, welcher beispielsweise in Spülpausen und/oder nachts oder am Wochenende durchgeführt werden kann. Dieser Stillstand kann derart ausgestaltet sein, dass lediglich die Beaufschlagung des Reinigungsguts mit dem Reinigungsfluid gestoppt ist, wobei jedoch eine oder mehrere weitere Verbraucher der Reinigungsvorrichtung nach wie vor aktiv sein können, wie beispielsweise eine Transportvorrichtung und/oder ein Gebläse. Alternativ kann der Stillstand auch derart ausgestaltet sein, dass sämtliche Verbraucher der Reinigungsvorrichtung im Stillstand und/oder in einem Standby-Zustand sind, in welchem keine Leistungsaufnahme durch diese Verbraucher und/oder lediglich eine minimale Leistungsaufnahme durch diese Verbraucher erfolgt. Während dieses Stillstands kann beispielsweise eine Aufladung des Energiespeichers mit aus dem elektrischen Anschluss aufgenommener elektrischer Energie erfolgen.

Der zweite genannte Betriebszustand, in welchem eine Entnahme elektrischer Energie aus dem Energiespeicher und eine Versorgung des mindestens einen Verbrauchers mit der Energie erfolgt, kann derart erfolgen, dass der Verbraucher ausschließlich aus dem Energiespeicher versorgt wird oder, alternativ, dass zusätzlich elektrische Energie, die über den elektrischen Anschluss aufgenommen wird, dem Verbraucher zugeführt wird. So kann die Reinigungsvorrichtung eingerichtet sein, um in dem zweiten Betriebszustand zusätzlich elektrische Energie über den elektrischen Anschluss aufzunehmen und dem elektrischen Verbraucher, zusätzlich zu einer Versorgung aus dem Energiespeicher, zuzuführen. Auf diese Weise kann, wie unten noch näher ausgeführt wird, beispielsweise eine maximale Leistungsaufnahme über den elektrischen Anschluss reduziert werden, sodass beispielsweise die maximale elektrische Leistungsaufnahme der Reinigungsvorrichtung im Vergleich zu einer Reinigungsvorrichtung mit identischen Verbrauchern, jedoch ohne Energiespeicher, reduziert sein kann, beispielsweise um mindestens 10%, vorzugsweise um mindestens 20% oder mehr.

Insbesondere kann die Reinigungsvorrichtung eingerichtet sein, um eine über den elektrischen Anschluss aufgenommene elektrische Leistung auf eine maximale Leistung zu begrenzen. Diese Leistungsbegrenzung kann beispielsweise durch mindestens eine entsprechende Überwachungsvorrichtung erfolgen, welche eine aktuelle Leistungsaufnahme erfasst, und/oder über mindestens eine Begrenzungsvorrichtung, welche eine aktuelle Leistungsaufnahme auf eine maximale Leistungsaufnahme begrenzt. Diesbezüglich kann beispielsweise auf den oben genannten Stand der Technik, insbesondere auf die DE 10 2009 035 668 A1, verwiesen werden. Die Reinigungsvorrichtung kann insbesondere derart eingerichtet sein, dass, bei identischem Betrieb, und der Option einer Versorgung des elektrischen Verbrauchers sowohl mit Energie aus dem Energiespeicher als auch mit über den elektrischen Anschluss direkt aufgenommener Energie die Leistungsaufnahme auf eine vorgegebene maximale Leistung begrenzt wird, welche geringer ist als eine maximale elektrische Leistung einer identischen Reinigungsvorrichtung ohne Energiespeicher.

Die Reinigungsvorrichtung kann weiterhin eingerichtet sein, um zumindest zeitweise eine Differenz zwischen der vorgegebenen maximalen Leistung und einer aktuell von dem Verbraucher insgesamt benötigten Leistung in dem Energiespeicher zwischenzuspeichern, insbesondere sofern eine aktuell von dem Verbraucher insgesamt benötigte Leistung geringer ist als die maximale Leistung. Diese Speicherung der Energiedifferenz kann beispielsweise dauerhaft oder lediglich während einer oder mehrerer Ladephasen erfolgen, während derer der Energiespeicher geladen werden kann.

Weitere mögliche Ausgestaltungen der Erfindung betreffen Ausgestaltungen des Energiespeichers. So kann der Energiespeicher beispielsweise eine Kapazität zur Zwischenspeicherung einer Energie von mindestens 10 Wh und bevorzugt von mindestens 100 Wh oder mindestens 1kWh, oder besonders bevorzugt von mindestens 10 kWh oder sogar mindestens 30 kWh.

Wie oben ausgeführt und wie unten noch näher erläutert, kann diese Energie beispielsweise in elektrischer Form oder auch in nicht-elektrischer Form gespeichert werden. Insbesondere kann der Energiespeicher eingerichtet sein, um die genannten Energiemengen für einen Zeitraum von mehreren Minuten, vorzugsweise von mindestens 10 Minuten, bevorzugt von mindestens 1 Stunde und besonders bevorzugt von mindestens 10 Stunden zwischenzuspeichern.

Wie oben ausgeführt, kann der Energiespeicher, wobei ein oder mehrere Energiespeicher vorgesehen sein können, als Ganzes oder in Teilen zur Zwischenspeicherung elektrischer Energie und/oder einer oder mehrerer anderer Energieformen eingerichtet sein. Sind mehre Energiespeicher vorgesehen, so können auch beispielsweise ein oder mehrere Energiespeicher zur Zwischenspeicherung elektrischer Energie eingerichtet sein und ein oder mehrere Energiespeicher zur Zwischenspeicherung einer oder mehrerer anderer Energieformen.

Insbesondere kann der Energiespeicher also mindestens einen elektrischen Energiespeicher aufweisen. Dieser elektrische Energiespeicher kann insbesondere ausgewählt sein aus der Gruppe bestehend aus: einem Akkumulator, insbesondere einem Blei-Säure-Akkumulator, einem NiCd-Akkumulator, einem NiMH-Akkumulator oder einem Lithium-Ionen-Akkumulator; einem Kondensator, insbesondere einem sogenannten Superkondensator oder "Supercap". Unter einem Supercap oder auch Superkondensator ist dabei allgemein im Rahmen der vorliegenden Erfindung ein Doppelschichtkondensator mit hoher Kapazität zu verstehen, welcher auf einer Dissoziation von Ionen in einem flüssigen Elektrolyten basiert, die ein Dielektrikum von wenigen Atomlagen und einer großen Elektrodenoberfläche bilden. Beispielsweise können Supercaps eine Energiedichte von mindestens 0,5 Wh/kg aufweisen, von mindestens 1 Wh/kg, insbesondere von mindestens 4 Wh/kg. Derartige Supercaps sind in verschiedenen Ausführungen kommerziell erhältlich. Auch Kombinationen der genannten elektrischen Energiespeicher und/oder anderer Arten von Energiespeichern sind grundsätzlich einsetzbar. Der elektrische Energiespeicher kann mindestens ein Energiewandler umfassen, welcher eingerichtet ist, um die über den elektrischen Anschluss aufgenommene elektrische Energie zunächst umzuwandeln, bevor diese in Form von elektrischer Energie zwischengespeichert wird. Beispielsweise kann dieser mindestens eine Energiewandler mindestens einen Gleichrichter und/oder mindestens einen Transformator umfassen. Weiterhin kann, alternativ oder zusätzlich, der elektrische Energiespeicher mindestens einen elektrischen Energiewandler aufweisen, welcher die in dem elektrischen Energiespeicher gespeicherte elektrische Energie vor Bereitstellung an den mindestens einen elektrischen Verbraucher umwandelt, beispielsweise einen Wechselrichter und/oder einen Transformator.

Wie oben ausgeführt, kann der Energiespeicher, alternativ oder zusätzlich zu mindestens einem elektrischen Energiespeicher, weiterhin mindestens einen nicht-elektrischen Energiespeicher umfassen. Auch in diesem Fall kann der Energiespeicher optional mindestens einen Energiewandler umfassen, wobei der Energiewandler eingerichtet sein kann, um eine Umwandlung zwischen elektrischer Energie und mindestens einer nicht-elektrischen Energieform vorzunehmen, wobei die nicht-elektrische Energieform in dem nicht-elektrischen Energiespeicher speicherbar ist. So kann beispielsweise der nicht-elektrische Energiespeicher mindestens einen ersten Energiewandler umfassen, welcher eingerichtet ist, um die über den elektrischen Anschluss aufgenommene elektrische Energie in mindestens eine nicht-elektrische Energieform umzuwandeln, die in dem nicht-elektrischen Energiespeicher speicherbar ist. Alternativ oder zusätzlich kann der Energiespeicher mindestens einen zweiten Energiewandler umfassen, welcher eingerichtet ist, um die nicht-elektrische Energieform, welche in dem nicht-elektrischen Energiespeicher gespeichert ist, vor Bereitstellung an den mindestens einen Verbraucher in elektrische Energie umzuwandeln.

Die Ausgestaltung des mindestens einen optionalen Energiewandlers kann insbesondere von der nicht-elektrischen Energieform abhängig sein. So kann der Energiewandler beispielsweise einen mechanisch-elektrischen Energiewandler und/oder einen thermisch-elektrischen Energiewandler und/oder einen chemisch-elektrischen Energiewandler aufweisen. Ein thermisch-elektrischer Energiewandler kann beispielsweise ein Heizelement und/oder ein Peltier-Element und/oder ein Seebeck-Element umfassen. Ein mechanisch-elektrischer Energiewandler kann beispielsweise einen Elektromotor und/oder einen Dynamo und/oder einen Generator umfassen. Ein chemischelektrischer Energiewandler kann beispielsweise mindestens eine Elektrolysezelle und/oder mindestens eine Brennstoffzelle umfassen.

In einer möglichen Ausgestaltung kann der nicht-elektrische Energiespeicher beispielsweise mindestens einen Wärmespeicher umfassen. Dieser Wärmespeicher kann beispielsweise mindestens ein Element mit einer spezifischen Wärmekapazität cₚ oder c_{V} von mindestens 1000 J/(kg · K), vorzugsweise von mindestens 2000 J/(kg · K), insbesondere von mindestens 4000 J/(kg · K) und besonders bevorzugt von mindestens 5000 J/(kg · K) aufweisen. Beispielsweise kann der Wärmespeicher mindestens einen Block und/oder mindestens einen Vorrat mindestens eines Werkstoffs mit der genannten Wärmekapazität aufweisen, beispielsweise einen Kupferblock und/oder einen Wassertank.

Der Wärmespeicher kann ausgewählt sein aus der Gruppe bestehend aus einem direkten Wärmespeicher und einem Latentwärmespeicher. Unter einem direkten Wärmespeicher ist dabei ein Wärmespeicher zu verstehen, welcher Wärme aufnehmen und speichern kann, ohne dass hierbei eine Phasenumwandlung stattfindet. So kann beispielsweise mindestens ein direkter Wärmespeicher in Form mindestens eines Wassertanks, gefüllt mit Wasser, vorzugsweise mit einer thermischen Isolierung und/oder mit mindestens einer Wärmepumpe, vorgesehen sein. Alternativ oder zusätzlich kann der direkte Wännespeicher auch mindestens einen Block eines Werkstoffs zur Wärmeaufnahme aufweisen, vorzugsweise eines Werkstoffs mit der genannten Wärmekapazität. Beispielsweise kann ein Block von mindestens 5 kg, vorzugsweise von mindestens 10 kg und besonders bevorzugt mindestens 40 kg des genannten Werkstoffs vorliegen. Unter einem direkten Wärmespeicher ist allgemein ein Element zu verstehen, welches unmittelbar die genannte Wärme aufnehmen kann und in Form von Wärme speichern kann.

Alternativ oder zusätzlich zu einem direkten Wärmespeicher kann der Wärmespeicher auch mindestens einen Latentwärmespeicher aufweisen. Unter einem Latentwärmespeicher ist allgemein im Rahmen der vorliegenden Erfindung eine Vorrichtung zu verstehen, welche mindestens einen Werkstoff aufweist, der reversibel mindestens einen Phasenübergang durchlaufen kann. So kann der Latentwärmespeicher Wärme aufnehmen, einen Phasenübergang durchführen und auf diese Weise die Wärmeenergie, in der Regel als andere Energieform, zwischenspeichern, um dann erneut, in umgekehrter Richtung, den selben oder einen anderen Phasenübergang durchführen zu können und dabei die aufgenommene Wärme vollständig oder teilweise wieder abgeben zu können. Beispiele von Phasenübergängen sind Schmelzvorgänge, Lösungsvorgänge, Absorptionsvorgänge oder Kristallisationsvorgänge. Derartige Phasenwechselmaterialien, welche auch im Rahmen der vorliegenden Erfindung einsetzbar sind, sind beispielsweise Salzspeicher, wie insbesondere Natriumacetat-Trihydrat. So kann beispielsweise in Wasser das Salzhydrat bei einer Schmelztemperatur von 58 °C verflüssigt werden und auch bei tieferen Temperaturen als unterkühlte Schmelze in einem metastabilen Zustand vorliegen. Das Salz kann sich in seinem Kristallwasser lösen. Eine Kristallisation kann durch einen Kristallisationskeim ausgelöst werden, wobei Wärme freigesetzt werden kann. Umgekehrt kann eine Verflüssigung durch eine Erwärmung des Salzspeichers initiiert werden. Auch andere Arten von Lätentwärmespeichern sind jedoch grundsätzlich im Rahmen der vorliegenden Erfindung einsetzbar.

Der nicht-elektrische Energiespeicher kann, alternativ oder zusätzlich zu einem Wärmespeicher, mindestens einen mechanischen Energiespeicher umfassen. Der mechanische Energiespeicher kann beispielsweise ausgewählt sein aus der Gruppe bestehend aus einem Druckspeicher, insbesondere einem Gasdruckspeicher und/oder einem Federspeicher; einem Schwungradspeicher. Auch andere mechanische Energiespeicher sind einsetzbar.

Der nicht-elektrische Energiespeicher kann weiterhin, alternativ oder zusätzlich zu den oben genannten Optionen, mindestens einen chemischen Energiespeicher umfassen. Unter einem chemischen Energiespeicher wird allgemein ein Energiespeicher verstanden, welcher eingerichtet ist, um Energie aufzunehmen und durch diese aufgenommene Energie mindestens eine chemische Reaktion, vorzugsweise mindestens eine reversible chemische Reaktion, zu starten. Weiterhin kann der chemische Energiespeicher eingerichtet sein, um eine umgekehrte chemische Reaktion, beispielsweise eine chemische Rückreaktion, durchzuführen und dabei wiederum eine Energie freizusetzen, beispielsweise thermische Energie. Ein Beispiel eines chemischen Energiespeichers, welcher im Rahmen der vorliegenden Erfindung einsetzbar ist, ist ein Gasspeicher. Insbesondere kann es sich hierbei um einen Wasserstoffspeicher und/oder mindestens einen Sauerstoffspeicher handeln. Der Energiespeicher kann insbesondere mindestens eine Elektrolysezelle und/oder mindestens eine Brennstoffzelle als Energiewandler umfassen, wobei die genannten Elemente auch einzeln oder beide als Energiewandler vorgesehen sein können. So kann beispielsweise der Energiespeicher derart eingerichtet sein, dass dieser über den elektrischen Anschluss aufgenommene elektrische Energie einer Elektrolysezelle zuführt, wobei in der Elektrolysezelle mindestens eine Elektrolysereaktion, beispielsweise eine Elektrolyse von Wasser, stattfindet, wobei die dabei entstehenden Elektrolyseprodukte, beispielsweise Wasserstoff und/oder Sauerstoff, gespeichert werden können. Weiterhin kann der Energiespeicher mindestens einen zweiten Energiewandler umfassen in Form mindestens einer Brennstoffzelle, wobei mittels der Brennstoffzelle die Elektrolyseprodukte, beispielsweise Wasserstoff und/oder Sauerstoff, reagieren können, wobei Wärme und/oder elektrische Energie erzeugt wird, welche direkt oder nach weiterer Umwandlung in elektrische Energie dem elektrischen Verbraucher zugeführt werden kann. Die Elektrolysezelle und die Brennstoffzelle können getrennt ausgebildet sein, können jedoch auch ganz oder teilweise bauteilidentisch ausgestaltet sein.

Die vorgeschlagene Reinigungsvorrichtung und das vorgeschlagene Verfahren weisen gegenüber bekannten Vorrichtungen und Verfahren der genannten Art zahlreiche Vorteile auf. So kann eine Speicherung von Energie in dem Energiespeicher insbesondere dann erfolgen, wenn die Reinigungsvorrichtung nicht voll ausgelastet ist oder beispielsweise während Betriebsunterbrechungen und/oder Stillstandszeiten, insbesondere nachts. Während eines Betriebs der Reinigungsvorrichtung, beispielsweise bei einem Volllastbetrieb, kann dann die gespeicherte Energie aus dem Energiespeicher abgerufen werden. Dadurch lässt sich die Leistung, die den elektrischen Verbrauchern der Reinigungsvorrichtung zur Verfügung gestellt wird erhöhen, ohne dass die elektrische Gesamtleistung, welche über den elektrischen Anschluss beispielsweise aus dem bauseitigen elektrischen Netz aufgenommen wird, steigt, beispielsweise über eine vorgegebene maximale Leistung hinaus. Ein derartiger Betrieb lässt sich auch als Booster-Betrieb bezeichnen. Mit dieser erhöhten, den elektrischen Verbrauchern zugeführten Leistung lässt sich auch eine Gesamtleistung der Reinigungsvorrichtung erhöhen, um beispielsweise größere Geschwindigkeiten und dadurch größere Durchsätze bei Transport-Geschirrspülmaschinen zu erzielen.

Auch bei der Herstellung einer Betriebsbereitschaft der Reinigungsvorrichtung, beispielsweise eine Geschirrspülmaschine, kann der Energiespeicher vorteilhaft zum Einsatz kommen. So kann beispielsweise in einer Betriebspause, insbesondere nachts, der Energiespeicher aufgeladen werden. Nach Beendigung der Betriebspause, beispielsweise morgens nach einem Befüllen der Reinigungsvorrichtung mit Reinigungsfluid, kann bei vergleichsweise kleiner Leistungsaufnahme über den elektrischen Anschluss eine deutlich höhere Heizleistung in der Reinigungsvorrichtung erzeugt werden, beispielsweise zur schnelleren Aufheizung eines oder mehrerer Reinigungsfluide, um damit beispielsweise eine schnellere Betriebsbereitschaft herzustellen.

Die Nutzung des Energiespeichers und dessen Vorteile werden umso interessanter, je ungleichmäßiger der Betrieb der Reinigungsvorrichtung ist. So kann die Reinigungsvorrichtung beispielsweise eingerichtet sein, um mindestens 2, vorzugsweise mindestens 3, mindestens 4 oder mehr Reinigungsprogramme durchzuführen. Die Reinigungsprogramme können beispielsweise jeweils einen oder mehrere Programmschritte umfassen, welche nacheinander, gleichzeitig oder auch zeitlich überlappend durchgeführt werden können. So können die Reinigungsprogrammschritte beispielsweise unterschiedliche Arten der Beaufschlagung des Reinigungsguts mit Reinigungsfluid umfassen, beispielsweise eine Beaufschlagung mit unterschiedlichen Arten von Reinigungsfluiden, beispielsweise eine Beaufschlagung mit einer Reinigerlösung und eine Beaufschlagung mit einer Klarspülerlösung, sowie optional eine Beaufschlagung mit einer Desinfektionsmittellösung und optional einer Beaufschlagung mit Heißdampf. Weiterhin können die Programmschritte eine Beaufschlagung mit Reinigungsfluiden unterschiedlicher Temperatur beinhalten. Weiterhin können die Reinigungsprogrammschritte eine oder mehrere Trocknungsphasen und/oder eine oder mehrere Abtropfphasen und/oder eine oder mehrere Entleerungsphasen (beispielsweise zur Entleerung von Ausscheidungsgefäßen zur Aufnahme menschlicher oder tierischer Ausscheidungen) aufweisen.

Je komplexer der Reinigungsbetrieb ist und je unterschiedlicher die Programmschritte der Reinigungsprogramme sind, desto unterschiedlicher und ungleichmäßiger ist in der Regel auch die Leistungsaufnahme der beteiligten elektrischen Verbraucher, wodurch der Einsatz des Energiespeichers umso vorteilhafter wird. Beispielsweise lässt sich bei einer Geschirrspülmaschine in Form eines Programmautomaten nach jedem gespülten Korb mit Reinigungsgut, beispielsweise bei einer Programmzeit von 90 s, eine Stillstandszeit von 90 s oder mehr die Auslegung eines bauseitigen elektrischen Anschlusses auf 50 % des Nennwerts reduzieren. Auch Reinigungs- und Desinfektionsgräte weisen in der Regel einen stark ungleichmäßigen Betriebsverlauf auf. Beispielsweise dauert ein Spülprogramm in derartigen Reinigungs- und Desinfektionsgeräten in der Regel 5 Minuten, worauf dann in der Regel eine Stillstandsphase von 10 Minuten folgt. Durch den Einsatz des genannten Energiespeichers kann im Falle eines Reinigungs- und Desinfektionsgerätes der elektrische Anschluss beispielsweise von einem dreiphasigen Drehstrom auf einen einphasigen Wechselstrom umgestellt werden, was in der Regel eine deutliche Vereinfachung bedeutet und gleichzeitig den möglichen Einsatzbereich der Reinigungsvorrichtung erweitern kann. So kann dann beispielsweise eine Reinigungs- und Desinfektionsvorrichtung auch leicht in einem Privathaushalt, der zumeist nicht über Drehstromanschlüsse verfügt, zum Einsatz kommen.

Weiterhin lässt sich durch den Einsatz des Energiespeichers die Leistungsaufnahme der Reinigungsvorrichtung, beispielsweise einer Spülmaschine und/oder eines Reinigungs- und Desinfektionsgerätes, in der Schwankungsbreite verkleinern, bei gleichzeitig bleibendem mittlerem Wert. So können Lastspitzen beispielsweise nicht aus dem Netz versorgt werden, sondern aus dem Energiespeicher. Die aufgenommene elektrische Spitzenlast kann so gegenüber heutigen Werten reduziert werden.

Wie oben ausgeführt, bestehen zahlreiche verschiedene Möglichkeiten, den Energiespeicher zu realisieren, welche an die jeweiligen Gegebenheiten und Anforderungen angepasst werden können. So kann die Energie beispielsweise elektrisch, in Form von Wärme, insbesondere direkt und/oder latent, mechanisch oder chemisch gespeichert werden. Auch Kombinationen der genannten Möglichkeiten sind realisierbar. Als elektrischer Energiespeicher lassen sich beispielsweise Akkumulatoren in unterschiedlichen Bauarten und/oder Technologien einsetzen, beispielsweise die oben genannten Akkumulatoren. Alternativ oder zusätzlich lassen sich als elektrische Energiespeicher ein oder mehrere elektrische Kondensatoren einsetzen, beispielsweise Supercaps. Wärmespeicher lassen sich leicht in Form von Wassertanks, insbesondere mit Isolierung und ggf. mindestens einer Wärmepumpe, realisieren. Latentwärmespeicher in Form von Salzspeichern sind technisch einfach realisierbar, was deren weite Verbreitung in Form von Wärmekissen zeigt. Auch mechanische Speicher, wie beispielsweise Druckspeicher in Form von Gasdruck- und/oder Federspeichern, oder Schwungradspeicher lassen sich einfach realisieren. Chemische Speicher lassen sich durch den Einsatz von Elektrolysezellen und Brennstoffzellen ebenfalls mit gängigen Technologien zuverlässig, sicher und ohne größeren Volumenbedarf realisieren.

Bei den aus dem oben genannten Stand der Technik bekannten Konzepten zur Erhöhung der Energieeffizienz von Reinigungsvorrichtungen wird in der Regel bereits in der Reinigungsvorrichtung vorhandene Energie weiter genutzt oder erneut genutzt. So lassen sich beispielsweise Reinigungsvorrichtungen mit Speichersystemen oder Wärmerückgewinnungseinrichtungen betreiben. Derartige Einrichtungen sind auch im Rahmen der vorliegenden Erfindung grundsätzlich zusätzlich nutzbar. Mittels des beschriebenen Energiespeichers lässt sich jedoch insbesondere elektrische Energie, bevor diese zum ersten Mal in der Reinigungsvorrichtung zum Einsatz kommt, Zwischenspeichern. Diese Zwischenspeicherung kann beispielsweise, wie oben ausgeführt, zur schnelleren Herstellung einer Betriebsbereitschaft oder zur Senkung einer Maximalleistung nutzen.

Insgesamt werden im Rahmen der vorliegenden Erfindung folgende Ausführungsformen als besonders bevorzugt betrachtet:
Ausführungsform 1: Reinigungsvorrichtung zum Reinigen von Reinigungsgut, wobei die Reinigungsvorrichtung mindestens eine Fluidvorrichtung zum Beaufschlagen des Reinigungsguts mit mindestens einem Reinigungsfluid aufweist, wobei die Reinigungsvorrichtung mindestens einen elektrischen Verbraucher aufweist, wobei die Reinigungsvorrichtung mindestens eine Reinigungskammer aufweist, in welcher die Beaufschlagung des Reinigungsguts mit dem Reinigungsfluid erfolgt, wobei die Reinigungsvorrichtung mindestens einen elektrischen Anschluss zur Versorgung der Reinigungsvorrichtung mit elektrischer Energie aufweist, wobei die Reinigungsvorrichtung mindestens einen Energiespeicher aufweist, wobei die Reinigungsvorrichtung eingerichtet ist, um über den elektrischen Anschluss elektrische Energie aufzunehmen und in dem Energiespeicher zwischenzuspeichern und wobei die Reinigungsvorrichtung weiterhin eingerichtet ist, um den mindestens einen elektrischen Verbraucher mit elektrischer Energie aus dem Energiespeicher zu versorgen, wobei die Reinigungsvorrichtung eingerichtet ist, um den mindestens einen elektrischen Verbraucher wahlweise mit elektrischer Energie aus dem Energiespeicher und wahlweise direkt mit über den elektrischen Anschluss aufgenommener elektrischer Energie zu versorgen, wobei die Reinigungsvorrichtung eingerichtet ist, um den mindestens einen elektrischen Verbraucher gleichzeitig mit elektrischer Energie aus dem Energiespeicher und direkt mit über den elektrischen Anschluss aufgenommener elektrischer Energie zu versorgen.

Ausführungsform 2: Reinigungsvorrichtung nach der vorhergehenden Ausführungsform, wobei die Reinigungsvorrichtung eingerichtet ist, um ein Verhältnis zwischen der elektrischen Energie aus dem Energiespeicher und der direkt über den elektrischen Anschluss aufgenommenen elektrischen Energie, mit der der elektrische Verbraucher beaufschlagt wird, zu verändern.

Ausführungsform 3: Reinigungsvorrichtung nach der vorhergehenden Ausführungsform wobei die Reinigungsvorrichtung eingerichtet ist, um das Verhältnis an einen aktuellen Betriebszustand anzupassen.

Ausführungsform 4: Reinigungsvorrichtung nach einer der vorhergehenden Ausführungsformen, wobei die Reinigungsvorrichtung eingerichtet ist, um eine über den elektrischen Anschluss aufgenommene elektrische Leistung auf eine vorgegebene maximale Leistung zu begrenzen.

Ausführungsform 5: Reinigungsvorrichtung nach der vorhergehenden Ausführungsform, wobei die Reinigungsvorrichtung eingerichtet ist, um eine Differenz zwischen der vorgegebenen maximalen Leistung und einer aktuell von dem Verbraucher insgesamt benötigten Leistung in dem Energiespeicher zwischenzuspeichern.

Ausführungsform 6: Reinigungsvorrichtung nach einer der vorhergehenden Ausführungsformen, wobei der Energiespeicher eine Kapazität zur Zwischenspeicherung einer Energie von mindestens 10 Wh aufweist und bevorzugt von mindestens 100 Wh oder mindestens 1kWh, oder besonders bevorzugt von mindestens 10 kWh oder sogar mindestens 30 kWh.

Ausführungsform 7: Reinigungsvorrichtung nach einer der vorhergehenden Ausführungsformen, wobei die Reinigungsvorrichtung ausgewählt ist aus der Gruppe bestehend aus: einer Geschirrspülmaschine, insbesondere einer Einkammer-Geschirrspülmaschine und/oder einer Durchlaufgeschirrspülmaschine; einem Reinigungs- und Desinfektionsgerät zur Reinigung von Ausscheidungsgefäßen zur Aufnahme menschlicher Ausscheidungen.

Ausführungsform 8: Reinigungsvorrichtung nach einer der vorhergehenden Ausführungsformen, wobei die Reinigungsvorrichtung eingerichtet ist, um in mindestens zwei verschiedenen Betriebszuständen betrieben zu werden, wobei in den Betriebszuständen der Verbraucher eine unterschiedliche elektrische Leistungsaufnahme aufweist, wobei in mindestens einem ersten Betriebszustand elektrische Energie über den elektrischen Anschluss aufgenommen wird und der Energiespeicher mit elektrischer Energie aufgeladen wird und wobei in mindestens einem zweiten Betriebszustand elektrische Energie aus dem Energiespeicher entnommen und dem elektrischen Verbraucher zugeführt wird.

Ausführungsform 9: Reinigungsvorrichtung nach der vorhergehenden Ausführungsform, wobei der erste Betriebszustand einen Stillstand der Reinigungsvorrichtung umfasst, wobei in dem Stillstand keine Beaufschlagung des Reinigungsguts mit dem Reinigungsfluid er-folgt.

Ausführungsform 10: Reinigungsvorrichtung nach einer der beiden vorhergehenden Ausführungsformen, wobei die Reinigungsvorrichtung eingerichtet ist, um in dem zweiten Betriebszustand zusätzlich elektrische Energie über den elektrischen Anschluss aufzunehmen und dem elektrischen Verbraucher zusätzlich zuzuführen.

Ausführungsform 11: Reinigungsvorrichtung nach einer der vorhergehenden Ausführungsformen, wobei der Energiespeicher mindestens einen elektrischen Energiespeicher aufweist.

Ausführungsform 12: Reinigungsvorrichtung nach der vorhergehenden Ausführungsform, wobei der elektrische Energiespeicher ausgewählt ist aus der Gruppe bestehend aus: einem Akkumulator, insbesondere einem Blei-Säure-Akkumulator, einem NiCd-Akkumulator, einem NiMH-Akkumulator oder einem Li-Ionen-Akkumulator; einem Kondensator, insbesondere einem Supercap.

Ausführungsform 13: Reinigungsvorrichtung nach einer der vorhergehenden Ausführungsformen, wobei der Energiespeicher weiterhin mindestens einen nicht-elektrischen Energiespeicher umfasst.

Ausführungsform 14: Reinigungsvorrichtung nach der vorhergehenden Ausführungsform, wobei der Energiespeicher mindestens einen Energiewandler umfasst, wobei der Energiewandler eingerichtet ist, um eine Umwandlung zwischen elektrischer Energie und mindestens einer nicht-elektrischen Energieform vorzunehmen, wobei die nicht-elektrische Energieform in dem nicht-elektrischen Energiespeicher speicherbar ist.

Ausführungsform 15: Reinigungsvorrichtung nach einer der beiden vorhergehenden Ausführungsformen, wobei der nicht-elektrische Energiespeicher mindestens einen Wärmespeicher umfasst.

Ausführungsform 16: Reinigungsvorrichtung nach der vorhergehenden Ausführungsform, wobei der Wärmespeicher ausgewählt ist aus der Gruppe bestehend aus: einem direkten Wärmespeicher, insbesondere einem Wassertank, vorzugsweise mit mindestens einer thermischen Isolierung und/oder mindestens einer Wärmepumpe; einem Latentwärmespeicher, insbesondere einem Salzspeicher.

Ausführungsform 17: Reinigungsvorrichtung nach einer der vier vorhergehenden Ausführungsformen, wobei der nicht-elektrische Energiespeicher mindestens einen mechanischen Energiespeicher umfasst.

Ausführungsform 18: Reinigungsvorrichtung nach der vorhergehenden Ausführungsform, wobei der mechanische Energiespeicher ausgewählt ist aus der Gruppe bestehend aus: einem Druckspeicher, insbesondere einem Gasdruckspeicher und/oder einem Federspeicher; einem Schwungradspeicher.

Ausführungsform 19: Reinigungsvorrichtung nach einer der sechs vorhergehenden Ausführungsformen, wobei der nicht-elektrische Energiespeicher mindestens einen chemischen Energiespeicher umfasst.

Ausführungsform 20: Reinigungsvorrichtung nach der vorhergehenden Ausführungsform, wobei der chemische Energiespeicher mindestens einen Gasspeicher umfasst, insbesondere mindestens einen Wasserstoffspeicher und/oder mindestens einen Sauerstoffspeicher.

Ausführungsform 21: Reinigungsvorrichtung nach einer der beiden vorhergehenden Ausführungsformen, wobei der Energiespeicher mindestens eine Elektrolysezelle und/oder mindestens eine Brennstoffzelle als Energiewandler umfasst.

Ausführungsform 22: Reinigungsvorrichtung nach einer der vorhergehenden Ausführungsformen, wobei der elektrische Verbraucher ausgewählt ist aus der Gruppe bestehend aus: einer Heizvorrichtung zur Erwärmung des mindestens einen Reinigungsfluids; einer Pumpe zur Förderung des mindestens einen Reinigungsfluids; einem Dampferzeuger zur Erzeugung von Heißdampf; einem Gebläse zur Förderung von mindestens einem Dampf und/oder mindestens einem Gas; einer Transportvorrichtung zur Beförderung des Reinigungsguts.

Ausführungsform 23: Verfahren zum Reinigen von Reinigungsgut, wobei das Reinigungsgut mittels mindestens einer Fluidvorrichtung mit mindestens einem Reinigungsfluid beaufschlagt wird, wobei bei dem Verfahren mindestens ein elektrischer Verbraucher verwendet wird, wobei mindestens ein elektrischer Anschluss zur Versorgung des Verfahrens mit elektrischer Energie verwendet wird, wobei mindestens ein Energiespeicher verwendet wird, wobei über den elektrischen Anschluss elektrische Energie aufgenommen wird und in dem

Energiespeicher zwischengespeichert wird, wobei der mindestens eine elektrische Verbraucher mit elektrischer Energie aus dem Energiespeicher versorgt wird, wobei eine Reinigungsvorrichtung nach einem der vorhergehenden Ausführungsformen verwendet wird.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1: ein erstes Ausführungsbeispiel einer erfindungsgemäßen Reinigungsvorrichtung in Form einer Einkammer-Geschirrspülmaschine;
- Figur 2: ein zweites Ausführungsbeispiel einer erfindungsgemäßen Reinigungsvorrichtung in Form einer Durchlaufgeschirrspülmaschine; und
- Figur 3: ein weiteres Ausführungsbeispiel einer erfindungsgemäßen Reinigungsvorrichtung in Form eines Reinigungs- und Desinfektionsgerätes.

### Ausführungsbeispiele

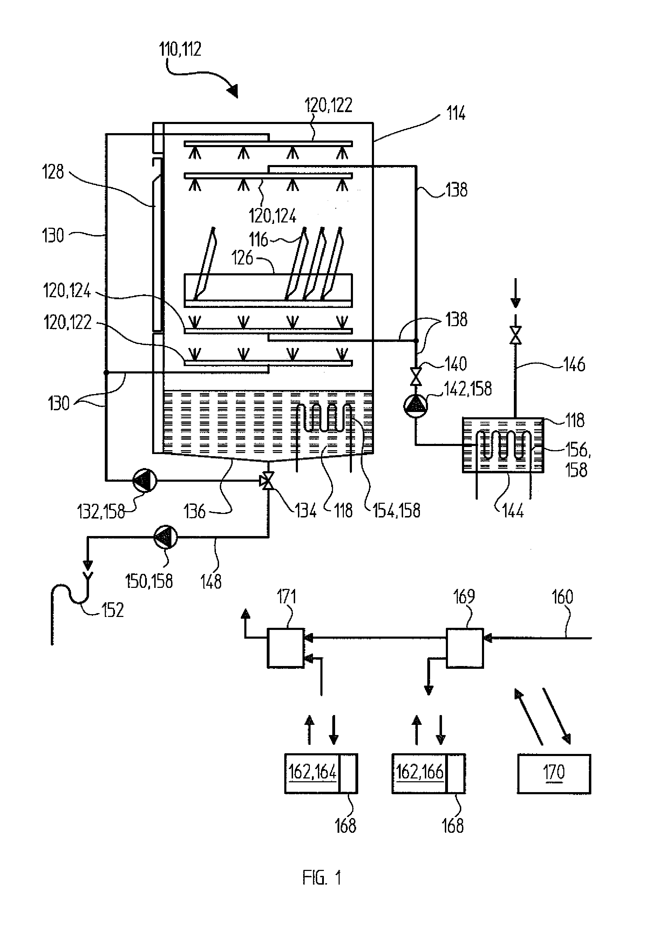

In Figur 1 ist ein erstes Ausführungsbeispiel einer erfindungsgemäßen Reinigungsvorrichtung 110 in einer schematischen Schnittdarstellung gezeigt. Die Reinigungsvorrichtung 110 ist in diesem Fall exemplarisch als Einkammer-Geschirrspülmaschine 112 ausgestaltet. Für Beispiele möglicher Ausgestaltungen derartiger Einkammer-Geschirrspülmaschinen 112 kann exemplarisch auf die DE 10 2004 046 758 A1 verwiesen werden. Auch andere Ausgestaltungen sind jedoch grundsätzlich möglich.

Die Reinigungsvorrichtung 110 umfasst in dem dargestellten Ausführungsbeispiel eine Reinigungskammer 114, in welcher Reinigungsgut 116, beispielsweise Geschirr, mit Reinigungsfluid 118 beaufschlagt wird. Für diese Beaufschlagung können ein oder mehrere Fluidvorrichtungen 120 vorgesehen sein, welche in dem dargestellten Ausführungsbeispiel exemplarisch ein Spüldüsensystem 122 und ein Nachspüldüsensystem 124 umfassen kann. Die Düsensysteme 122, 124 können beispielsweise innerhalb der Reinigungskammer 114 oberhalb und unterhalb eines Korbs 126 angeordnet sein, in dem das Reinigungsgut 116 aufgenommen ist. Die Beladung der Reinigungskammer 114 mit dem Reinigungsgut 116 kann beispielsweise über eine Tür 128, beispielsweise eine Frontklappe, erfolgen. Das Spüldüsensystem 122 kann beispielsweise über Spülleitungen 130, eine Spülpumpe 132 und über ein 3-Wege-Ventil 134 mit Reinigungsfluid 118, beispielsweise einer Reinigerlösung, aus einem Spültank 136 gespeist werden, welcher sich beispielsweise im Bodenbereich der Reinigungskammer 114 befinden kann. Das optionale Nachspüldüsensystem 124 kann beispielsweise über Nachspülleitungen 138, ein Nachspülventil 140 und eine Nachspülpumpe 142 mit Reinigungsfluid 118 aus einem Nachspültank 144 beaufschlagt werden, beispielsweise mit Nachspülfluid in Form einer Klarspülerlösung. Der Nachspültank 144 kann beispielsweise mit Frischwasser über eine Frischwasserzuleitung 146 gespeist werden. Weiterhin kann die Reinigungsvorrichtung 110 eine Ablaufleitung 148 aufweisen, welche beispielsweise mit dem Spültank 136 über das 3-Wege-Ventil 134 verbunden ist, welche optional eine Ablaufpumpe 150 umfassen kann und welche optional mit einem Abfluss 152 verbunden sein kann.

Die Reinigungsvorrichtung 110 kann weiterhin mindestens ein Heizelement 154 zur Erwärmung des Reinigungsfluids 118 in dem Spültank 136 aufweisen. Weiterhin kann die Reinigungsvorrichtung 110 ein weiteres Heizelement 156 in dem Nachspültank 144 zur Erwärmung des dort aufgenommenen Reinigungsfluids 118 in Form des Nachspülfluids aufweisen. Beispielsweise kann der Nachspültank 144 als Boiler ausgestaltet sein und/oder kann einen Durchlauferhitzer aufweisen oder mit einem Durchlauferhitzer verbunden sein.

In der Reinigungsvorrichtung 110, welche beispielsweise als Programmautomat ausgestaltet sein kann, kann beispielsweise ein Reinigungsprogramm ablaufen. In diesem Reinigungsprogramm kann beispielsweise ein erster Programmschritt durchgeführt werden, bei welchem eine Spülung des Reinigungsguts 116 aus dem Spültank 136 erfolgt. Diese Spülung kann beispielsweise in einem Umwälzbetrieb über die Spülpumpe 132, welche auch als Umwälzpumpe bezeichnet werden kann, erfolgen. Anschließend kann über die Ablaufpumpe 150 und das 3-Wege-Ventil 134 die Spülflüssigkeit teilweise oder vollständig aus dem Spültank 136 abgeleitet werden. Parallel zu dem Spülschritt kann bereits zuvor in dem Nachspültank 144 eine Aufbereitung, beispielsweise eine Erwärmung, von Nachspülfluid erfolgt sein. In einem dem Spülschritt nachgelagerten weiteren Programmschritt kann anschließend eine Nachspülung oder Klarspülung des Reinigungsguts 116 mit Reinigungsfluid aus den Nachspültank 144 erfolgen, was in einem Einfachdurchlauf oder, optional ebenfalls in einem Umwälzbetrieb erfolgen kann. Ein oder mehrere weitere Programmschritte können folgen, beispielsweise ein oder mehrere Trocknungsschritte, bevor das Reinigungsprogramm beendet werden kann.

Die Reinigungsvorrichtung 110 gemäß dem in Figur 1 dargestellten Ausführungsbeispiel umfasst mehrere elektrische Verbraucher 158. Beispielsweise sind hierbei insbesondere die Heizelemente 154, 156 zu nennen. Weitere Ausführungsbeispiele in dem in Figur 1 gezeigten Beispiel sind die Pumpen 132, 142 und 150.

Zur Versorgung dieser elektrischen Verbraucher mit elektrischer Energie ist die Reinigungsvorrichtung 110 mit einem elektrischen Anschluss 160 verbunden. Weiterhin weist die Reinigungsvorrichtung 110 erfindungsgemäß einen oder mehrere Energiespeicher 162 auf. Beispielsweise sind in Figur 1 optional ein elektrischer Energiespeicher 164 und/oder ein nicht-elektrischer Energiespeicher 166 dargestellt, welche jeweils über einen oder mehrere Energiewandler 168 verfügen können. Wie oben beschrieben, kann in dem Energiespeicher 162 über den elektrischen Anschluss 160 aufgenommene elektrische Leistung in Form von elektrischer und/oder nicht-elektrischer Energie zwischengespeichert werden, bevor diese allen, einzelnen oder mehreren der elektrischen Verbraucher 158 zugeführt wird. Für mögliche Ausgestaltungen der Energiespeicher 162 kann auf die obige Beschreibung verwiesen werden.

Die Reinigungsvorrichtung 110 kann weiterhin mindestens eine Steuerung 170 umfassen, welche zentral oder auch dezentral ausgestaltet sein kann. Diese Steuerung 170 kann beispielsweise mindestens eine Datenverarbeitungsvorrichtung umfassen. Die Steuerung 170 kann beispielsweise eingerichtet sein, um ein oder mehrere Reinigungsprogramme zu steuern und kann zu diesem Zweck beispielsweise die elektrischen Verbraucher 158 entsprechend ansteuern. Die Steuerung 170 kann beispielsweise mindestens eine Benutzerschnittstelle und/oder mindestens eine Schnittstelle zu einem anderen Gerät umfassen, über welche Informationen und/oder Befehle unidirektional oder bidirektional austauschbar sind.

Die Steuerung 170 kann weiterhin die Verteilung von Energieflüssen innerhalb der Reinigungsvorrichtung 110 steuern. Beispielsweise kann die Steuerung 170 mit mindestens einer Messvorrichtung verbunden sein, welche in Figur 1 nicht dargestellt ist, und welche einen aktuellen Leistungsbedarf und/oder eine aktuelle Leistungsaufnahme bestimmen kann. Dementsprechend kann die Steuerung 170 beispielsweise variabel die Energiespeicher 162 einzeln, in Gruppen oder insgesamt aufladen durch über den elektrischen Anschluss 160 aufgenommene elektrische Energie und/oder kann die Energiespeicher 162 entladen, wobei in den Energiespeichern 162 gespeicherte Energie einem, mehreren, oder allen der elektrischen Verbraucher 158 zugewiesen wird.

Zu diesem Zweck kann die Reinigungsvorrichtung 110 in diesem oder auch in anderen Ausführungsbeispielen eines oder mehrere Schaltelemente aufweisen, welche in Figur 1 mit den Bezugsziffern 169 und 171 bezeichnet sind. Beispielsweise kann mindestens ein optionales Schaltelement 169 vorgesehen sein, welches einstellt, beispielsweise gesteuert durch die Steuerung 170, welcher Anteil der aus dem elektrischen Anschluss 160 aufgenommenen elektrischen Leistung, ohne Zwischenspeicherung in dem Energiespeicher 162, den elektrischen Verbrauchern 158 zugeführt wird und welcher Anteil der aus dem elektrischen Anschluss 160 aufgenommenen elektrischen Leistung in dem Energiespeicher 162 zwischengespeichert wird. Die Einstellung dieser Anteile kann digital oder analog erfolgen und/oder kann, wie oben beschrieben, stufenlos oder in zwei oder mehreren Stufen erfolgen. Alternativ oder zusätzlich kann mindestens ein Schaltelement 171 vorgesehen sein, welches beispielsweise einstellen kann, wiederum beispielsweise gesteuert durch die Steuerung 170, zu welchem Anteil aktuell die elektrischen Verbraucher 158 mit elektrischer Energie aus dem Energiespeicher 162 versorgt werden und zu welchem Anteil die elektrischen Verbraucher 158 mit elektrischer Energie direkt aus dem elektrischen Anschluss 160, ohne Zwischenspeicherung in dem Energiespeicher 162, versorgt werden. Auch die Einstellung dieser Anteile kann digital oder analog erfolgen und/oder kann, wie oben beschrieben, stufenlos oder in zwei oder mehreren Stufen erfolgen. Das mindestens eine Schaltelement 171 kann auch ganz oder teilweise bauteilidentisch mit dem mindestens einen Schaltelement 169 ausgestaltet sein. Weiterhin können die Schaltelemente 169, 171 und die Energiespeicher 164, 166 getrennt ausgebildet sein oder auch ganz oder teilweise zusammengefasst sein. Weiterhin können die Schaltelemente 169, 171 für die verschiedenen Verbraucher 158 getrennt ausgebildet sein, oder es kann mindestens eines der Schaltelemente 169, 171 für mehrere Verbraucher 158 oder sogar für alle der Verbraucher 158 gemeinsam vorgesehen sein. Die Schaltelemente können beispielsweise ein oder mehrere Relais, elektronische Relais, Schütze, Halbleiter-Schütze, Leistungstransistoren und/oder andere Arten von Schaltelementen umfassen.

Beispielsweise kann die Steuerung eingerichtet sein, um in einer Betriebsphase eines Stillstands der Reinigungsvorrichtung 110 die Energiespeicher 162 einzeln, zu mehreren oder insgesamt aufzuladen. Weiterhin kann die Steuerung 170 eingerichtet sein, um in mindestens einer Betriebsphase, in welcher eine Beaufschlagung des Reinigungsguts 116 mit Reinigungsfluid 118 erfolgt, im Energiespeicher 162 gespeicherte Energie, optional nach Umwandlung durch die Energiewandler 168, den elektrischen Verbrauchern 158 zuzuführen, alternativ oder zusätzlich zu einer elektrischen Energie, welche unmittelbar über den elektrischen Anschluss 160 aufgenommen wird. Für weitere mögliche Ausgestaltungen kann auf die obige Beschreibung verwiesen werden.

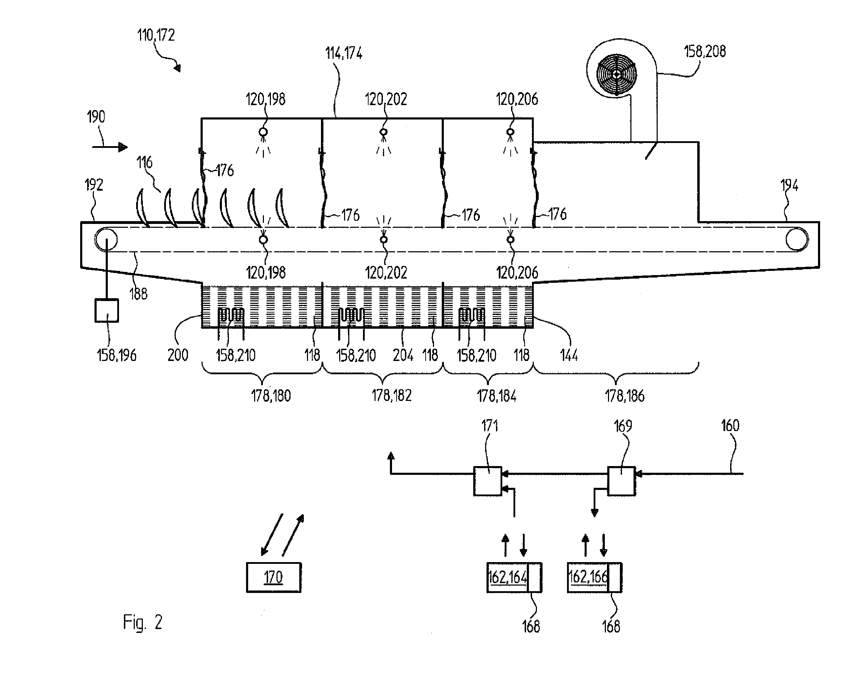

In Figur 2 ist ein weiteres Ausführungsbeispiel einer erfindungsgemäßen Reinigungsvorrichtung 110 in einer Schnittdarstellung von der Seite gezeigt. In diesem Ausführungsbeispiel handelt es sich bei der Reinigungsvorrichtung 110 um eine Durchlaufgeschirrspülmaschine 172. Für mögliche Ausgestaltungen derartiger Durchlaufgeschirrspülmaschinen, die erfindungsgemäß abgewandelt und/oder ergänzt werden können, kann beispielsweise auf die oben zitierten DE 10 2004 046 758 A1, DE 10 2007 053 381 B3 oder DE 10 2009 035 668 A1 verwiesen werden. Auch andere Ausgestaltungen sind jedoch grundsätzlich möglich.

Die Reinigungsvorrichtung 110 weist in dem dargestellten Ausführungsbeispiel wiederum eine Reinigungskammer 114 in Form eines Tunnels 174 auf. Der Tunnel 174 ist durch Trennvorhänge 176 in mehrere Zonen 178 unterteilt, wobei in dem dargestellten Ausführungsbeispiel exemplarisch eine Vorabräumzone 180, eine Spülzone 182, eine Klarspülzone 184 und eine Trocknungszone 186 vorgesehen sind. Auch eine andere Ausgestaltung der Zonen 178 ist möglich.

In dem dargestellten Ausführungsbeispiel wird Reinigungsgut 116, beispielsweise wieder in Form von Geschirr, mittels mindestens einer Transportvorrichtung 188 in einer Transportrichtung 190 durch den Tunnel 174 befördert. Zu diesem Zweck kann die Reinigungsvorrichtung 110 beispielsweise als Bandtransportmaschine oder als Korbtransportmaschine ausgestaltet sein. Beispielsweise kann mindestens ein Einlauf 192 vorgesehen sein, an welchem das Reinigungsgut 116 auf die Transportvorrichtung 188 aufgegeben wird, und mindestens ein Auslauf 194, an welchem das gereinigte Reinigungsgut 116 entnommen werden kann. Die Transportvorrichtung 188 kann dementsprechend beispielsweise ein Förderband umfassen. Auch andere Ausgestaltungen sind möglich. Die Transportvorrichtung 188 kann beispielsweise mindestens einen Antrieb 196 umfassen.

In den Zonen 180, 182 und 184 wird das Reinigungsgut 116 wiederum über Fluidvorrichtungen 120 mit Reinigungsfluid 118 beaufschlagt. Beispielsweise kann zu diesem Zweck in der Vorabräumzone 180 ein Vorabräumzonen-Düsensystem 198 vorgesehen sein, welches, beispielsweise über eine in Figur 2 nicht dargestellte Pumpe und ein nicht dargestelltes Leitungssystem aus einem Vorabräumtank 200 mit Reinigungsfluid gespeist werden kann. In der Spülzone 182 kann beispielsweise ein Spülzonen-Düsensystem 202 vorgesehen sein, welches, beispielsweise über eine ebenfalls nicht dargestellte Pumpe und ein ebenfalls nicht dargestelltes Leitungssystem, aus einem Spültank 204 mit Reinigungsfluid 118 gespeist werden kann. In der Klarspülzone 184, welche einteilig oder auch mehrteilig ausgestaltet sein kann, kann beispielsweise ein Nachspüldüsensystem 206 vorgesehen sein, welches mit erwärmtem Frischwasser und/oder mit Nachspülfluid aus einem Nachspültank 144 gespeist werden kann.

Nach Durchlaufen der Zonen 180, 182 und 184 kann das Reinigungsgut 116 dann in die Trocknungszone 186 eintreten, in welcher das Reinigungsgut beispielsweise über ein Gebläse 208 mit Warmluft beaufschlagt werden kann, um die Trocknung des Reinigungsguts 116 zu beschleunigen.

Wiederum weist die Reinigungsvorrichtung 110 in dem dargestellten Ausführungsbeispiel mehrere elektrische Verbraucher 158 auf. Beispielsweise können in den Tanks 200, 204 und 144, einzeln oder in Gruppen, Heizelemente 210 vorgesehen sein. Weiterhin können als elektrische Verbraucher 158 in dem dargestellten Ausführungsbeispiel der Antrieb 196 der Transportvorrichtung 188 und das Gebläse 208 angesehen werden, wobei das Gebläse 208 optional als weiteren elektrischen Verbraucher 158 eine Heizvorrichtung umfassen kann. Weiterhin können die in Figur 2 nicht dargestellten Pumpen zur Beaufschlagung der Fluidvorrichtungen 120 als elektrische Verbraucher 158 fungieren.

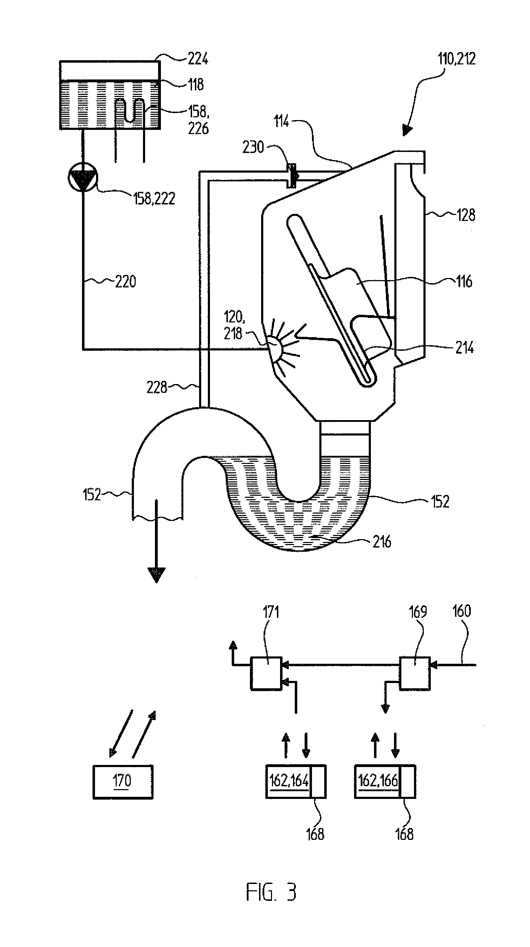

Zur Realisierung der oben ausgeführten Erfindung kann auch die Reinigungsvorrichtung 110 in dem dargestellten Ausführungsbeispiel gemäß Figur 2 wiederum einen oder mehrere Energiespeicher 162 umfassen, beispielsweise wiederum in Form von elektrischen Energiespeichern 164 und/oder nicht-elektrischen Energiespeichern 166. So kann beispielsweise elektrische Leistung über einen elektrischen Anschluss 160 aufgenommen werden und diese in dem mindestens einen Energiespeicher 162 zwischengespeichert werden oder, optional, wahlweise direkt den elektrischen Verbrauchern 158 zugeführt werden. Wiederum kann eine Steuerung 170 vorgesehen sein, welche beispielsweise verschiedene Betriebszustände der Reinigungsvorrichtung 110 einstellen kann und welche beispielsweise eine Verteilung der elektrischen Energie und die Durchführung eines erfindungsgemäßen Verfahrens gewährleisten kann. Zu diesem Zweck können beispielsweise wiederum ein oder mehrere Schaltelemente 169, 171 vorgesehen sein, wobei wiederum auf die obige Beschreibung verwiesen werden kann. Beispielsweise können während längerer Stillstandszeiten der Reinigungsvorrichtung 110 einer oder mehrere der Energiespeicher 162 aufgeladen werden, beispielsweise nachts. Die dort zwischengespeicherte Energie kann bei einem Anfahren der Maschine und einer Überführung der Reinigungsvorrichtung 110 in einen Betriebszustand, in welchem das Reinigungsgut 110 mit dem Reinigungsfluid 118 beaufschlagt wird, einem, mehreren, oder allen der elektrischen Verbraucher 158 zugeführt werden. Wie oben ausgeführt, kann auf diese Weise beispielsweise eine Herstellung einer Betriebsbereitschaft der Reinigungsvorrichtung 110 beschleunigt werden und/oder es kann eine insgesamt aufgenommenne maximale Leistung gegenüber herkömmlicher Reinigungsvorrichtungen 110 gesenkt werden. In Figur 3 ist ein drittes Ausführungsbeispiel einer Reinigungsvorrichtung 110 in einer Schnittdarstellung von der Seite gezeigt. In diesem Ausführungsbeispiel ist die Reinigungsvorrichtung 110 als Reinigungs- und Desinfektionsgerät 212 ausgestaltet. Wiederum umfasst die Reinigungsvorrichtung 110 eine Reinigungskammer 114 mit einer Tür 128, beispielsweise einer Frontklappe. In der Reinigungskammer 114 kann Reinigungsgut 116, beispielsweise in Form von einem oder mehreren Ausscheidungsgefäßen zur Aufnahme größerer Mengen menschlicher oder tiereischer Ausscheidungen aufgenommen sein. Dieses Reinigungsgut 116 kann beispielsweise in einer entsprechenden Halterung 214 gehaltert sein. Vorzugsweise ist diese Halterung 214 derart ausgestaltet, dass, beim Schließen der Tür 128, das Reinigungsgut 116 automatisch in einen Abfluss 152, welcher insbesondere einen Siphonbogen oder eine andere Art der Geruchssperre umfassen kann, entleert wird.

In der Reinigungskammer 114 kann das Reinigungsgut 116 mit Reinigungsfluid 118 beaufschlagt werden. Zu diesem Zweck kann beispielsweise wiederum eine Fluidvorrichtung 120 in Form eines Düsensystems 218 vorgesehen sein. Dieses Düsensystem 218 kann beispielsweise über ein Leitungssystem 220 und optional eine Pumpe 222 aus einem oder mehreren Tanks 224 gespeist werden. Hierbei können auch nacheinander unterschiedliche Arten von Reinigungsfluids 118 zum Einsatz kommen. Beispielsweise kann zunächst, beispielsweise nach Entleerung in den Abfluss 152, eine Beaufschlagung mit kalter, wässriger Reinigungsflüssigkeit erfolgen, dann in einem weiteren Schritt eine Beaufschlagung mit erwärmter, wässriger Reinigungsflüssigkeit erfolgen, ggf. unter Zusatz eines oder mehrerer Reinigungsmittel und/oder eines oder mehrerer Desinfektionsmittel. Anschließend kann optional eine Beaufschlagung mit Heißdampf erfolgen, zu welchem Zweck die Reinigungsvorrichtung 110 beispielsweise einen in Figur 3 nicht dargestellten Dampferzeuger umfassen kann. Die Beaufschlagung mit unterschiedlichen Reinigungsfluiden 118 kann über ein und dasselbe Fluidsystem 120 oder auch über verschiedene Fluidsysteme erfolgen. Zur Erzeugung von erwärmtem Reinigungsfluid 118 und/oder zur Erzeugung von Heißdampf können wiederum ein oder mehrere Heizelemente 226 vorgesehen sein.

Nach Beaufschlagung des Reinigungsguts 116 mit dem Reinigungsfluid 118 kann sich eine Trocknungsphase anschließen. Während dieser Trocknungsphase kann beispielsweise Frischluft oder auch Heißluft in die Reinigungskammer 114 eingebracht werden, beispielsweise zwangsweise über ein in Figur 3 nicht dargestelltes Gebläse. Bei dieser zwangsweisen Einbringung von Frischluft oder erwärmter Luft kann die in der Reinigungskammer 114 vorhandene feuchte, Luft und/oder Dampf über einen Bypass 228, optional mit einem Rückschlagventil 230, unter Umgehung der Geruchssperre bzw. des Siphonbogens 216 in den Abfluss 252 verdrängt werden. Auf diese Weise kann sicher gestellt werden, dass bei Öffnen der Tür 128 keine feuchte Luft und kein heißer Dampf in die Umgebung gelangen kann.

Wiederum kann die Reinigungsvorrichtung 110 gemäß dem Ausführungsbeispiel in Figur 3 eine Mehrzahl von elektrischen Verbrauchern 158 umfassen. Beispielsweise können in Figur 3 die Pumpe 222 und/oder das Heizelement 226 als elektrische Verbraucher 158 ausgestaltet sein. Zur Versorgung dieser elektrischen Verbraucher 158 mit elektrischer Energie wird wiederum über einen elektrischen Anschluss 160 von der Reinigungsvorrichtung 110 elektrische Energie aufgenommen. Wiederum sind erfindungsgemäß ein oder mehrere Energiespeicher 162 vorgesehen, optional wiederum mit einem oder mehreren Energiewandlern 168, in welchen Energie, die über den elektrischen Anschluss 160 aufgenommen wurde, vor Zuführung an einen elektrischen Verbraucher 158 zwischengespeichert werden kann. Dementsprechend kann eine Beaufschlagung der elektrischen Verbraucher 158 mit Energie aus dem Energiespeicher 162 erfolgen und/oder, alternativ oder zusätzlich, direkt mit über den elektrischen Anschluss 160 aufgenommener elektrische Energie ohne Zwischenspeicherung. Wiederum kann eine Steuerung 170 vorgesehen sein, um, beispielsweise entsprechend einem Programmablauf und einem aktuellen Betriebszustand der Reinigungsvorrichtung, die Verteilung der elektrischen Energie zu steuern. Wiederum können ein oder mehrere Schaltelemente 169, 171 vorgesehen sein, bezüglich deren möglicher Ausgestaltung auf die obige Beschreibung verwiesen werden kann.

**Bezugszeichenliste**

| | | | |
|---|---|---|---|
| 110 | Reinigungsvorrichtung | 174 | Tunnel |
| 112 | Einkammer-Geschirrspülmaschine | 176 | Trennvorhang |
| 114 | Reinigungskammer | 178 | Zone |
| 116 | Reinigungsgut | 180 | Vorabräumzone |
| 118 | Reinigungsfluid | 182 | Spülzone |
| 120 | Fluidvorrichtung | 184 | Klarspülzone |
| 122 | Spüldüsensystem | 186 | Trocknungszone |
| 124 | Nachspüldüsensystem | 188 | Transportvorrichtung |
| 126 | Korb | 190 | Transportrichtung |
| 128 | Tür | 192 | Einlauf |
| 130 | Spülleitungen | 194 | Auslauf |
| 132 | Spülpumpe | 196 | Antrieb |
| 134 | 3-Wege-Ventil | 198 | Vorabräumzone-Düsensystem |
| 136 | Spültank | 200 | Vorabräumtank |
| 138 | Nachspülleitungen | 202 | Spülzonen-Düsensystem |
| 140 | Nachspülventil | 204 | Spültank |
| 142 | Nachspülpumpe | 206 | Nachspüldüsensystem |
| 144 | Nachspültank | 208 | Gebläse |
| 146 | Frischwasserzuleitung | 210 | Heizelement |
| 148 | Ablaufleitung | 212 | Reinigungs- und Desinfektionsge-rät |
| 150 | Ablaufpumpe | | |
| 152 | Abfluss | 214 | Halterung |
| 154 | Heizelement | 216 | Siphonbogen |
| 156 | Heizelement | 218 | Düsensystem |
| 158 | elektrischer Verbraucher | 220 | Leitungssystem |
| 160 | Elektrischer Anschluss | 222 | Pumpe |
| 162 | Energiespeicher | 224 | Tank |
| 164 | elektrischer Energiespeicher | 226 | Heizelement |
| 166 | nicht-elektrischer Energiespeicher | 228 | Bypass |
| 168 | Energiewandler | 230 | Rückschlagventil |
| 169 | Schaltelement | | |
| 170 | Steuerung | | |
| 171 | Schaltelement | | |
| 172 | Durchlaufgeschirrspülmaschine | | |

## Patentansprüche

1. Reinigungsvorrichtung (110) zum Reinigen von Reinigungsgut (116), wobei die Reinigungsvorrichtung (110) mindestens eine Fluidvorrichtung (120) zum Beaufschlagen des Reinigungsguts (116) mit mindestens einem Reinigungsfluid (118) aufweist, wobei die Reinigungsvorrichtung (110) mindestens eine Reinigungskammer (114) aufweist, in welcher die Beaufschlagung des Reinigungsguts (116) mit dem Reinigungsfluid erfolgt, wobei die Reinigungsvorrichtung (110) mindestens einen elektrischen Verbraucher (158) aufweist, wobei die Reinigungsvorrichtung (110) mindestens einen elektrischen Anschluss (160) zur Versorgung der Reinigungsvorrichtung (110) mit elektrischer Energie aufweist, wobei die Reinigungsvorrichtung (110) mindestens einen Energiespeicher (162) aufweist, wobei die Reinigungsvorrichtung (110) eingerichtet ist, um über den elektrischen Anschluss (160) elektrische Energie aufzunehmen und in dem Energiespeicher (162) zwischenzuspeichern und wobei die Reinigungsvorrichtung (110) weiterhin eingerichtet ist, um den mindestens einen elektrischen Verbraucher (158) mit elektrischer Energie aus dem Energiespeicher (162) zu versorgen, **dadurch gekennzeichnet, dass** die Reinigungsvorrichtung (110) eingerichtet ist, um den mindestens einen elektrischen Verbraucher (158) wahlweise mit elektrischer Energie aus dem Energiespeicher (162) und wahlweise direkt mit über den elektrischen Anschluss (160) aufgenommener elektrischer Energie zu versorgen, wobei die Reinigungsvorrichtung (110) eingerichtet ist, um den mindestens einen elektrischen Verbraucher (115) gleichzeitig mit elektrischer Energie aus dem Energiespeicher (162) und direkt mit über den elektrischen Anschluss (160) aufgenommener elektrischer Energie zu versorgen.

2. Reinigungsvorrichtung (110) nach dem vorhergehenden Anspruch, wobei die Reinigungsvorrichtung (110) eingerichtet ist, um ein Verhältnis zwischen der elektrischen Energie aus dem Energiespeicher (162) und der direkt über den elektrischen Anschluss (160) aufgenommenen elektrischen Energie, mit der der elektrische Verbraucher (115) beaufschlagt wird, zu verändern.

3. Reinigungsvorrichtung (110) nach dem vorhergehenden Anspruch, wobei die Reinigungsvorrichtung (110) eingerichtet ist, um das Verhältnis an einen aktuellen Betriebszustand anzupassen.

4. Reinigungsvorrichtung (110) nach einem der vorhergehenden Ansprüche, wobei die Reinigungsvorrichtung (110) eingerichtet ist, um eine über den elektrischen Anschluss (160) aufgenommene elektrische Leistung auf eine vorgegebene maximale Leistung zu begrenzen.

5. Reinigungsvorrichtung (110) nach dem vorhergehenden Anspruch, wobei die Reinigungsvorrichtung (110) eingerichtet ist, um eine Differenz zwischen der vorgegebenen maximalen Leistung und einer aktuell von dem Verbraucher (115) insgesamt benötigten Leistung in dem Energiespeicher (162) zwischenzuspeichern.

6. Reinigungsvorrichtung (110) nach einem der vorhergehenden Ansprüche, wobei der Energiespeicher (162) eine Kapazität zur Zwischenspeicherung einer Energie von mindestens 10 Wh aufweist.

7. Reinigungsvorrichtung (110) nach einem der vorhergehenden Ansprüche, wobei die Reinigungsvorrichtung (110) ausgewählt ist aus der Gruppe bestehend aus: einer Geschirrspülmaschine, insbesondere einem Programmautomaten (112) und/oder einer Durchlaufgeschirrspülmaschine (172); einem Reinigungs- und Desinfektionsgerät (212).

8. Reinigungsvorrichtung (110) nach einem der vorhergehenden Ansprüche, wobei die Reinigungsvorrichtung (110) eingerichtet ist, um in mindestens zwei verschiedenen Betriebszuständen betrieben zu werden, wobei in den Betriebszuständen der Verbraucher (158) eine unterschiedliche elektrische Leistungsaufnahme aufweist, wobei in mindestens einem ersten Betriebszustand elektrische Energie über den elektrischen Anschluss (160) aufgenommen wird und der Energiespeicher (162) mit elektrischer Energie aufgeladen wird und wobei in mindestens einem zweiten Betriebszustand elektrische Energie aus dem Energiespeicher (162) entnommen und dem elektrischen Verbraucher (158) zugeführt wird.

9. Reinigungsvorrichtung (110) nach dem vorhergehenden Anspruch, wobei der erste Betriebszustand einen Stillstand der Reinigungsvorrichtung (110) umfasst, wobei in dem Stillstand keine Beaufschlagung des Reinigungsguts (116) mit dem Reinigungsfluid (118) erfolgt.

10. Reinigungsvorrichtung (110) nach einem der vorhergehenden Ansprüche, wobei der Energiespeicher (162) mindestens einen elektrischen Energiespeicher (164) aufweist.

11. Reinigungsvorrichtung (110) nach dem vorhergehenden Anspruch, wobei der elektrische Energiespeicher (164) ausgewählt ist aus der Gruppe bestehend aus: einem Akkumulator, insbesondere einem Blei-Säure-Akkumulator, einem NiCd-Akkumulator, einem NiMH-Akkumulator oder einem Li-Ionen-Akkumulator; einem Kondensator, insbesondere einem Supercap.

12. Reinigungsvorrichtung (110) nach einem der vorhergehenden Ansprüche, wobei der Energiespeicher (162) weiterhin mindestens einen nicht-elektrischen Energiespeicher (166) umfasst.

13. Reinigungsvorrichtung (110) nach dem vorhergehenden Anspruch, wobei der Energiespeicher (162) mindestens einen Energiewandler (168) umfasst, wobei der Energiewandler eingerichtet ist, um eine Umwandlung zwischen elektrischer Energie und mindestens einer nicht-elektrischen Energieform vorzunehmen, wobei die nicht-elektrische Energieform in dem nicht-elektrischen Energiespeicher (166) speicherbar ist.

14. Reinigungsvorrichtung (110) nach einem der beiden vorhergehenden Ansprüche, wobei der nicht-elektrische Energiespeicher (166) mindestens einen Wärmespeicher umfasst.

15. Verfahren zum Reinigen von Reinigungsgut (116), wobei das Reinigungsgut (116) mittels mindestens einer Fluidvorrichtung (120) mit mindestens einem Reinigungsfluid (118) beaufschlagt wird, wobei bei dem Verfahren mindestens ein elektrischer Verbraucher (158) verwendet wird, wobei mindestens ein elektrischer Anschluss (160) zur Versorgung des Verfahrens mit elektrischer Energie verwendet wird, wobei mindestens ein Energiespeicher (162) verwendet wird, wobei über den elektrischen Anschluss (160) elektrische Energie aufgenommen wird und in dem Energiespeicher (162) zwischengespeichert wird, wobei der mindestens eine elektrische Verbraucher (158) mit elektrischer Energie aus dem Energiespeicher (162) versorgt wird, wobei eine Reinigungsvorrichtung (110) nach einem der vorhergehenden Ansprüche verwendet wird.

## Claims

1. Cleaning device (110) for cleaning items (116) to be cleaned, wherein the cleaning device (110) has at least one fluid device (120) for applying at least one cleaning fluid (118) to the items (116) to be cleaned, wherein the cleaning device (110) has at least one cleaning chamber (114) in which the cleaning fluid is applied to the items (116) to be cleaned, wherein the cleaning device (110) has at least one electrical load (158), wherein the cleaning device (110) has at least one electrical connection (160) for supplying electrical energy to the cleaning device (110), wherein the cleaning device (110) has at least one energy store (162), wherein the cleaning device (110) is designed to receive electrical energy via the electrical connection (160) and to temporarily store said electrical energy in the energy store (162), and wherein the cleaning device (110) is further designed to supply electrical energy from the energy store (162) to the at least one electrical load (158), **characterized in that** the cleaning device (110) is designed to selectively supply electrical energy from the energy store (162) and selectively directly supply electrical energy which is received via the electrical connection (160) to the at least one electrical load (158), wherein the cleaning device (110) is designed to simultaneously supply electrical energy from the energy store (162) and directly supply electrical energy which is received via the electrical connection (160) to the at least one electrical load (115).

2. Cleaning device (110) according to the preceding claim, wherein the cleaning device (110) is designed to change a ratio between the electrical energy from the energy store (162) and the electrical energy which is received directly via the electrical connection (160), in which ratio said electrical energy is applied to the electrical load (115).

3. Cleaning device (110) according to the preceding claim, wherein the cleaning device (110) is designed to match the ratio to a current operating state.

4. Cleaning device (110) according to one of the preceding claims, wherein the cleaning device (110) is designed to limit an electrical power which is received via the electrical connection (160) to a prespecified maximum power.

5. Cleaning device (110) according to the preceding claim, wherein the cleaning device (110) is designed to temporarily store in the energy store (162) a difference between the prespecified maximum power and a power which is currently required in total by the load (115).

6. Cleaning device (110) according to one of the preceding claims, wherein the energy store (162) has a capacity for temporarily storing an energy of at least 10 Wh.

7. Cleaning device (110) according to one of the preceding claims, wherein the cleaning device (110) is selected from the group consisting of: a dishwasher, in particular a batch dishwasher (112) and/or a conveyor-type dishwasher (172); a cleaning and disinfection apparatus (212).

8. Cleaning device (110) according to one of the preceding claims, wherein the cleaning device (110) is designed to be operated in at least two different operating states, wherein the load (158) has a different electrical power consumption in the operating states, wherein electrical energy is received via the electrical connection (160) and the energy store (162) is charged with electrical energy in at least a first operating state, and wherein electrical energy is drawn from the energy store (162) and supplied to the electrical load (158) in at least a second operating state.

9. Cleaning device (110) according to the preceding claim, wherein the first operating state comprises a downtime of the cleaning device (110), wherein no cleaning fluid (118) is applied to the items (116) to be cleaned during the downtime.

10. Cleaning device (110) according to one of the preceding claims, wherein the energy store (162) has at least one electrical energy store (164).

11. Cleaning device (110) according to the preceding claim, wherein the electrical energy store (164) is selected from the group consisting of: a rechargeable battery, in particular a lead-acid rechargeable battery, an NiCd rechargeable battery, an NiMH rechargeable battery or a lithium-ion rechargeable battery; a capacitor, in particular a supercap.

12. Cleaning device (110) according to one of the preceding claims, wherein the energy store (162) further comprises at least one non-electrical energy store (166).

13. Cleaning device (110) according to the preceding claim, wherein the energy store (162) comprises at least one energy converter (168), wherein the energy converter is designed to convert between electrical energy and at least one non-electrical form of energy, wherein the non-electrical form of energy can be stored in the non-electrical energy store (166).

14. Cleaning device (110) according to either of the two preceding claims, wherein the non-electrical energy store (166) comprises at least one heat store.

15. Method for cleaning items (116) to be cleaned, wherein at least one cleaning fluid (118) is applied to the items (116) to be cleaned by means of at least one fluid device (120), wherein at least one electrical load (158) is used in the method, wherein at least one electrical connection (160) is used for supplying electrical energy to the method, wherein at least one energy store (162) is used, wherein electrical energy is received via the electrical connection (160) and said electrical energy is temporarily stored in the energy store (162), wherein electrical energy from the energy store (162) is supplied to the at least one electrical load (158), wherein a cleaning device (110) according to one of the preceding claims is used.

## Revendications

1. Dispositif de nettoyage (110) destiné à nettoyer un produit à nettoyer (116), le dispositif de nettoyage (110) possédant au moins un dispositif à fluide (120) destiné à exposer le produit à nettoyer (116) à au moins un fluide de nettoyage (118), le dispositif de nettoyage (110) possédant au moins une chambre de nettoyage (114) dans laquelle a lieu l'exposition du produit à nettoyer (116) au fluide de nettoyage, le dispositif de nettoyage (110) possédant au moins un consommateur électrique (158), le dispositif de nettoyage (110) possédant au moins une borne électrique (160) servant à l'alimentation du dispositif de nettoyage (110) en énergie électrique, le dispositif de nettoyage (110) possédant au moins un accumulateur d'énergie (162), le dispositif de nettoyage (110) étant conçu pour collecter de l'énergie électrique par le biais de la borne électrique (160) et la stocker temporairement dans l'accumulateur d'énergie (162) et le dispositif de nettoyage (110) étant en outre conçu pour alimenter l'au moins un consommateur électrique (158) en énergie électrique depuis l'accumulateur d'énergie (162), **caractérisé en ce que** le dispositif de nettoyage (110) est conçu pour alimenter l'au moins un consommateur électrique (158), au choix, en énergie électrique en provenance de l'accumulateur d'énergie (162) et, au choix, directement avec l'énergie électrique collectée par le biais de la borne électrique (160), le dispositif de nettoyage (110) étant conçu pour alimenter l'au moins un consommateur électrique (115) simultanément avec de l'énergie électrique en provenance de l'accumulateur d'énergie (162) et directement avec l'énergie électrique collectée par le biais de la borne électrique (160).

2. Dispositif de nettoyage (110) selon la revendication précédente, avec lequel le dispositif de nettoyage (110) est conçu pour modifier un rapport entre l'énergie électrique en provenance de l'accumulateur d'énergie (162) et directement avec l'énergie électrique collectée par le biais de la borne électrique (160), avec lequel est alimenté le consommateur électrique (115).

3. Dispositif de nettoyage (110) selon la revendication précédente, avec lequel le dispositif de nettoyage (110) est conçu pour adapter le rapport à un état de fonctionnement actuel.

4. Dispositif de nettoyage (110) selon l'une des revendications précédentes, avec lequel le dispositif de nettoyage (110) est conçu pour limiter une puissance électrique collectée par le biais de la borne électrique (160) à une puissance maximale prédéfinie.

5. Dispositif de nettoyage (110) selon la revendication précédente, avec lequel le dispositif de nettoyage (110) est conçu pour stocker temporairement dans l'accumulateur d'énergie (162) une différence entre la puissance maximale prédéfinir et une puissance totale requise actuellement par le consommateur (115).

6. Dispositif de nettoyage (110) selon l'une des revendications précédentes, avec lequel l'accumulateur d'énergie (162) possède une capacité de stockage temporaire d'une énergie d'au moins 10 Wh.

7. Dispositif de nettoyage (110) selon l'une des revendications précédentes, avec lequel le dispositif de nettoyage (110) est choisi dans le groupe composé de : un lave-vaisselle, notamment un appareil automatique programmable (112) et/ou un lave-vaisselle à passage (172) ; un appareil de nettoyage et de désinfection (212).

8. Dispositif de nettoyage (110) selon l'une des revendications précédentes, avec lequel le dispositif de nettoyage (110) est conçu pour fonctionner dans au moins deux états de fonctionnement différents, le consommateur (158) possédant une puissance consommée électrique différente dans les états de fonctionnement, dans au moins un premier état de fonctionnement de l'énergie électrique étant collectée par le biais de la borne électrique (160) et l'accumulateur d'énergie (162) étant chargé avec de l'énergie électrique, et dans au moins un deuxième état de fonctionnement de l'énergie électrique étant prélevée de l'accumulateur d'énergie (162) et acheminée au consommateur électrique (158).

9. Dispositif de nettoyage (110) selon la revendication précédente, avec lequel le premier état de fonctionnement comprend une immobilisation du dispositif de nettoyage (110), aucune exposition du produit à nettoyer (116) au fluide de nettoyage (118) n'ayant lieu dans l'état d'immobilisation.

10. Dispositif de nettoyage (110) selon l'une des revendications précédentes, avec lequel l'accumulateur d'énergie (162) possède au moins un accumulateur d'énergie électrique (164).

11. Dispositif de nettoyage (110) selon la revendication précédente, avec lequel l'accumulateur d'énergie électrique (164) est choisi dans le groupe composé de : un accumulateur, notamment un accumulateur au plomb-acide, un accumulateur au NiCd, un accumulateur au NiMH ou un accumulateur aux ions de Li ; un condensateur, notamment un supercondensateur.

12. Dispositif de nettoyage (110) selon l'une des revendications précédentes, avec lequel l'accumulateur d'énergie (162) comprend en outre au moins un accumulateur d'énergie non électrique (166).

13. Dispositif de nettoyage (110) selon la revendication précédente, avec lequel l'accumulateur d'énergie (162) comprend au moins un convertisseur d'énergie (168), le convertisseur d'énergie étant conçu pour effectuer une conversion entre l'énergie électrique et au moins un forme d'énergie non électrique, la forme d'énergie non électrique pouvant être stockée dans l'accumulateur d'énergie non électrique (166).

14. Dispositif de nettoyage (110) selon l'une des deux revendications précédentes, avec lequel l'accumulateur d'énergie non électrique (166) comprend au moins un accumulateur de chaleur.

15. Procédé de nettoyage d'un produit à nettoyer (116), le produit à nettoyer (116) étant exposé au moyen d'au moins un dispositif à fluide (120) à au moins un fluide de nettoyage (118), au moins un consommateur électrique (158) étant utilisé lors du procédé, au moins une borne électrique (160) servant à l'alimentation du procédé en énergie électrique étant utilisée, au moins un accumulateur d'énergie (162) étant utilisé, de l'énergie électrique étant collectée par le biais de la borne électrique (160) et étant stocke temporairement dans l'accumulateur d'énergie (162)au moins un consommateur électrique (158) étant alimenté en énergie électrique depuis l'accumulateur d'énergie (162), un dispositif de nettoyage (110) selon l'une des revendications précédentes étant utilisé.
